# EUROPEAN PATENT SPECIFICATION

(11) **EP 2 598 244 B1**
(45) Date of publication and mention of the grant of the patent: **01.10.2014**
(21) Application number: 11743317.7
(22) Date of filing: 21.07.2011
(51) Int. Cl.: B01L 3/00, G01N 33/543, G01N 35/00

(54) **SAMPLE PLATE**
PROBENPLATTE
PLAQUE D'ÉCHANTILLON

(30) Priority: 19.04.2011 GB 201106618; 25.01.2011 GB 201101222; 29.07.2010 WO PCT/GB2010/001443; 29.07.2010 US 846580
(43) Date of publication of application: 05.06.2013
(73) Proprietor: Dynex Technologies, Inc., Chantilly, VA 20151-1621 (US)
(72) Inventor: BUNCE, Adrian, Worthing West Sussex BN13 3DE (GB); FUSELLIER, Andrew, Bristow Virginia 20136 (US)
(74) Representative: Jeffrey, Philip Michael
(86) International application number: PCT/GB2011/051383
(87) International publication number: WO 2012/013959

(56) References cited:
- EP-A1- 2 279 790
- WO-A2-02/30561
- WO-A2-2004/111260
- WO-A2-2007/022026
- US-A- 4 681 742
- US-A- 5 620 853
- US-A1- 2003 186 228
- US-A1- 2009 069 200

## Description

### BACKGROUND TO THE INVENTION

The present invention relates to a sample plate.

An automated reagent bead or microsphere dispenser for dispensing reagent beads or microspheres into a sample plate is disclosed. The sample plate may be used to carry out diagnostic testing such as Enzyme Linked ImmunoSorbent Assay ("ELISA") procedures or other immunoassay procedures. Alternatively, the sample plate may be used to carry out testing for DNA or RNA sequences.

Immunoassay procedures are a preferred way of testing biological products. These procedures exploit the ability of antibodies produced by the body to recognise and combine with specific antigens which may, for example, be associated with foreign bodies such as bacteria or viruses, or with other body products such as hormones. Once a specific antigen-antibody combination has occurred it can be detected using chromogenic, fluorescent or chemiluminescent materials or less preferably by using radioactive substances. Radioactive substances are less preferred due to environmental and safety concerns regarding their handling, storage and disposal. The same principles can be used to detect or determine any materials which can form specific binding pairs, for example using lectins, rheumatoid factor, protein A or nucleic acids as one of the binding partners.

ELISA is a particularly preferred form of immunoassay procedure wherein one member of the binding pair Is linked to an insoluble carrier surface ("the solid phase") such as a sample vessel, and after reaction the bound pair is detected by use of a further specific binding agent conjugated to an enzyme ("the conjugate"). The procedures for ELISA are well known in the art and have been in use for both research and commercial purposes for many years. Numerous books and review articles describe the theory and practice of immunoassays. Advice is given, for example, on the characteristics and choice of solid phases for capture assays, on methods and reagents for coating solid phases with capture components, on the nature and choice of labels, and on methods for labelling components. An example of a standard textbook is "ELISA and Other Solid Phase Immunoassays, Theoretical and Practical Aspects", Editors D.M. Kemeny & S.J. Challacombe, published by John Wiley, 1988. Such advice may also be applied to assays for other specific binding pairs.

In the most common type of ELISA, the solid phase is coated with a member of the binding pair. An aliquot of the specimen to be examined is incubated with the solid coated solid phase and any analyte that may be present is captured onto the solid phase. After washing to remove residual specimen and any interfering materials it may contain, a second binding agent, specific for the analyte and conjugated to an enzyme is added to the solid phase. During a second incubation any analyte captured onto the solid phase will combine with the conjugate. After a second washing to remove any unbound conjugate, a chromogenic substrate for the enzyme is added to the solid phase. Any enzyme present will begin to convert the substrate to a chromophoric product. After a specified time the amount of product formed may be measured using a spectrophotometer, either directly or after stopping the reaction.

It will be realised that the above is an outline description of a general procedure for bioassay and that many variants are known in the art including fluorogenic and luminogenic substrates for ELISA, direct labelling of the second member of the binding pair with a fluorescent or luminescent molecule (in which case the procedure is not called an ELISA but the process steps are very similar) and nucleic acids or other specific pairing agents instead of antibodies as the binding agent. However, all assays require that fluid samples, e.g. blood, serum, urine, etc., are aspirated from a sample tube and are then dispensed into a solid phase. Samples may be diluted prior to being dispensed into the solid phase or they may be dispensed into deep well microplates, diluted in situ and then the diluted analyte may be transferred to the functional solid phase.

The most common type of solid phase is a standard sample vessel known as a microplate which can be stored easily and which may be used with a variety of biological specimens. Microplates have been available commercially since the 1960s and are made from e.g. polystyrene, PVC, Perspex or Lucite and measure approximately 5 inches (12.7 cm) in length, 3.3 inches (8.5 cm) in width, and 0.55 inches (1.4 cm) in depth. Microplates made from polystyrene are particularly preferred on account of polystyrene's enhanced optical clarity which assists visual interpretation of the results of any reaction. Polystyrene microplates are also compact, lightweight and easily washable. Microplates manufactured by the Applicants are sold under the name "MICROTITRE" (RTM). Known microplates comprise 96 wells (also commonly known as "microwells") which are symmetrically arranged in an 8 x 12 array. Microwells typically have a maximum volume capacity of approximately 350 µl. However, normally only 10-200 µl of fluid is dispensed into a microwell. In some arrangements of the microplate the microwells may be arranged in strips of 8 or 12 wells that can be moved and combined in a carrier to give a complete plate having conventional dimensions.

Positive and negative controls are generally supplied with commercial kits and are used for quality control and to provide a relative cut-off. After reading the processed microplate, the results of the controls are checked against the manufacturer's validated values to ensure that the analysis has operated correctly and then the value is used to distinguish positive from negative specimens and a cut-off value is calculated. Standards are usually provided for quantitative assays and are used to build a standard curve from which the concentration of analyte in a specimen may be interpolated.

It will be recognised that the ELISA procedure as outlined above involves multiple steps including pipetting, incubation, washing, transferring microplates between activities, reading and data analysis. In recent years systems have been developed which automate the steps (or "phases") involved in the ELISA procedures such as sample distribution, dilution, incubation at specific temperatures, washing, enzyme conjugate addition, reagent addition, reaction stopping and the analysis of results. The pipette mechanism used to aspirate and dispense fluid samples uses disposable tips which are ejected after being used so as to prevent cross-contamination of patients' samples. Multiple instrumental controls are in place to ensure that appropriate volumes, times, wavelengths and temperatures are employed, data transfer and analysis is fully validated and monitored. Automated immunoassay apparatus for carrying out ELISA procedures are now widely used in laboratories of e.g. pharmaceutical companies, veterinary and botanical laboratories, hospitals and universities for in-vitro diagnostic applications such as testing for diseases and infection, and for assisting in the production of new vaccines and drugs.

ELISA kits are commercially available which consist of microplates having microwells which have been coated by the manufacturer with a specific antibody (or antigen). For example, in the case of a hepatitis B antigen diagnostic kit, the kit manufacturer will dispense anti-hepatitis B antibodies which have been suspended in a fluid into the microwells of a microplate. The microplate is then incubated for a period of time, during which time the antibodies adhere to the walls of the microwells up to the fluid fill level (typically about half the maximum fluid capacity of the microwell). The microwells are then washed leaving a microplate having microwells whose walls are uniformly covered with anti-hepatitis B antibodies up to the fluid fill level.

A testing laboratory will receive a number of sample tubes containing, for example, body fluid from a number of patients. A specified amount of fluid is then aspirated out of the sample tube using a pipette mechanism and is then dispensed into one or more microwells of a microplate which has been previously prepared by the manufacturer as discussed above. If it is desired to test a patient for a number of different diseases then fluid from the patient must be dispensed into a number of separate microplates, each coated by its manufacturer with a different binding agent. Each microplate can then be processed separately to detect the presence of a different disease. It will be seen that to analyse several different analytes requires a multiplicity of microplates and transfer of aliquots of the same specimen to the different microplates. This leads to large numbers of processing steps and incubators and washing stations that can cope with many microplates virtually simultaneously. In automated systems this requires instruments to have multiple incubators and complex programming is required to avoid clashes between microplates with different requirements. For manual operation either several technicians are required or the throughput of specimens is slow. It is possible to combine strips of differently coated microwells into a single carrier, add aliquots of a single specimen to the different types of well and then perform the ELISA in this combined microplate. Constraints on assay development, however, make this combination difficult to achieve and it is known in the art that for users to combine strips in this fashion can lead to errors of assignment of result, while manufacture of microplates with several different coatings in different microwells presents difficulties of quality control.

Conventional ELISA techniques have concentrated upon performing the same single test upon a plurality of patient samples per microplate or in detecting the presence of one or more of a multiplicity of analytes in those patients without distinguishing which of the possible analytes is actually present. For example, it is commonplace to determine in a single microwell whether a patient has antibodies to HIV-1 or HIV-2, or HIV-1 or -2 antigens, without determining which analyte is present and similarly for HCV antibodies and antigens.

However, a new generation of assays are being developed which enable multiplexing to be performed. Multiplexing enables multiple different tests to be performed simultaneously upon the same patient sample.

A recent approach to multiplexing is to provide a microplate comprising 96 sample wells wherein an array of different capture antibodies is disposed in each sample well. The array comprises an array of 20 nl spots each having a diameter of 350 µm. The spots are arranged with a pitch spacing of 650 µm. Each spot corresponds with a different capture antibody.

Multiplexing enables a greater number of data points and more information per assay to be obtained compared with conventional ELISA techniques wherein each sample plate tests for a single analyte of interest. The ability to be able to combine multiple separate tests into the same assay can lead to considerable time and cost savings. Multiplexing also enables the overall footprint of the automated apparatus to be reduced.

Although there are many advantageous aspects to current known ELISA techniques and to the new multiplex techniques which are currently being developed, it is nonetheless desired to provide a sample plate and associated automated apparatus which has an improved format and which provides a greater flexibility than state of the art ELISA arrangements.

In addition to ELISA procedures it is also known to use a hybridization probe to test for the presence of DNA or RNA sequences. A hybridization probe typically comprises a fragment of DNA or RNA which is used to detect the presence of nucleotide sequences which are complementary to the DNA or RNA sequence on the probe. The hybridization probe hybridizes to single-stranded nucleic acid (e.g. DNA or RNA) whose base sequence allows pairing due to complementarity between the hybridization probe and the sample being analysed. The hybridization probe may be tagged or labelled with a molecular marker such as a radioactive or more preferably a fluorescent molecule. The probes are inactive until hybridization at which point there is a conformational change and the molecule complex becomes active and will then fluoresce (which can be detected under UV light) DNA sequences or RNA transcripts which have a moderate to high sequence similarity to the probe are then detected by visualising the probe under UV light.

An assay device and assembly for detecting an analyte in a liquid sample is disclosed in US-5620853 (Chiron Corporation). The assay device comprises a moulded well comprising fingers which protrude up from the bottom of the well and into which a reagent bead is dispensed. The reagent bead is captured in the fingers but can still move up and down within the finger height. The assay device is arranged to expose the reagent bead to as much fluid flow as possible and to rely upon signal from the underside of the reagent bead to produce results.

There are a number of problems with the arrangement disclosed in US-5620853.

Firstly, since the reagent beads are free to move up and down within the finger height then it is possible that a reagent bead may become stuck at an undesired height during a processing or reading step. In particular, the design of the well is relatively intricate and complex and any movement of, or damage to, the fingers could result in a reagent bead becoming stuck at an undesired height. The fingers also protrude from the base which makes them susceptible to damage particularly during pipetting and washing stages. If a reagent bead does become stuck at an undesired height within the fingers then this is highly likely to have an adverse effect upon the accuracy of the testing procedures.

Secondly, the design of the well with fingers which are arranged to receive a single reagent bead is such that fluid is pipetted next to the bead and the bead is covered by the rising fluid in the well. The single wells need approximately 300 µl of fluid. US-5620853 also discloses an arrangement wherein multiple wells are in fluid communication with each other. For the multi-well arrangement, each well will need approximately 300 µl of fluid. It will be apparent, therefore, that the multi-well arrangement requires an excessive amount of fluid to be dispensed relative to conventional systems.

Thirdly, the arrangement of fingers reduces the maximum packing density of wells for a given size sample plate so that relatively few tests can be performed on a given sample plate.

Fourthly, the multi-well arrangement disclosed in US-5620853 is particularly prone to crosstalk.

Fifthly, the arrangement disclosed in US-5620853 is such that when a single bead is used then the homogeneity of the fluid is only affected by the protruding fingers. There are likely to be regions of the well which will trap unmixed fluid. The multi-well arrangement also suffers from the serious problem that any fluid required to go over all beads has to pass through a tortuous path to get from one well to another. This will cause serious problems in terms of fluid mixing and bead to bead repeatability. The single well arrangement is completely different to the in-line multi-well arrangement disclosed in US-5620853 and the two different arrangements would therefore have quite different fluid characteristics. This is likely to result in different fluid behaviours depending upon the arrangement used and hence there is likely to be significant variation in results depending upon whether a single well or a multi-well format was used. Although in theory the two different arrangements could be validated independently, this would result in increased cost and reduced throughput.

Finally, the sample well disclosed in US-5620853 is relatively complex to manufacture and is likely to suffer from unreliability issues during manufacture. The long thin fingers are difficult to form by moulding and would be prone to damage during manufacture or during use. The fingers also have a feature at the top which in a mould tool would be an undercut. When the part is ejected off the tool the fingers must bend for the feature to get past the tool material. Such a manufacturing process is generally undesirable due to unreliability issues. Furthermore, any change in the process parameters is likely to affect the ability to release the part from the tool and leave the part intact to the correct mechanical tolerances. The position of the fingers relative to each other would be critical to allow the reagent bead to move up and down correctly and also to ensure that the reagent bead does not come out of the top of the fingers. This would be very difficult, in practice, to control in a mass production environment. It is also noted that the design of the single bead arrangement is completely different to the design of the multi-well arrangement. As a result, completely different tool designs would be required which again would greatly increase the complexity of manufacture. In a high volume manufacturing environment the combination of the design features and quality assurance concerns would make the sample plates excessively expensive to produce.

US 2009/0069200 (Yu) discloses a system for preparing arrays of biomolecules. According to the arrangement disclosed in US 2009/0069200 spherical beads are arranged within subwells which have a square cross-section. The spherical beads do not form a circumferential seal with the wall of the subwell and as a result fluid passes up from the bottom of the subwell, past the beads and over the top of the beads so that the beads are fully submerged or immersed. There are a number of problems with such an arrangement which are discussed in more detail later in the present application.

It is desired to provide an improved sample plate for retaining reagent beads.

### SUMMARY OF THE INVENTION

According to an aspect of the present Invention there is provided a sample plate as claimed in claim 1.

The one or more through holes pass from the bottom of the sample well to the rear or bottom surface of the sample plate. As a result, if a reagent bead is not retained or secured within the open through hole then any fluid in the sample well can leak out of the sample well via the through hole.

A sample plate is also disclosed comprising one or more sample wells, wherein one or more of the sample wells comprise:
a base portion; and
one or more recesses provided in the base portion;
characterised in that:
   a reagent bead or microsphere is substantially retained or secured, in use, within the recess so as to form a substantially fluid-tight circumferential seal with a wall of the base portion which defines the recess.

It should be understood that a circular bead within a hole, bore or recess having a square cross-section will not form a fluid-tight circumferential seal with the wall defining the hole, bore or recess. A fluid-tight circumferential seal should be understood as meaning that a barrier is formed around the entire circumference of the bead and the wall defining the hole, bore or recess.

According to the present invention reagent beads or microspheres are retained or secured within a through hole formed in the base portion of the sample plate. Each reagent bead or microsphere forms a fluid-tight and/or water-tight and/or air-tight seal about the entire outer diameter or circumference of the reagent bead or microsphere.

It will be understood that the spherical reagent beads in the arrangement disclosed in US 2009/0069200 do not form a fluid-tight circumferential seal with the square wall defining the subwell.

Once the reagent bead or microsphere is located within the through hole then fluid is substantially prevented from being able to pass from one side of the through hole to the other side by the reagent bead or microsphere which forms a tight seal about the entire circumference of the reagent bead or microsphere.

Various different embodiments are contemplated.

A blind recess differs from a through hole in that if a reagent bead is not retained or secured in a blind recess then sample fluid within a sample well will not leak out of the sample well.

Open through holes provided in the base portion of a sample well are substantially cylindrical and have a diameter less than a diameter of a reagent bead or microsphere deposited in the through hole so that the reagent bead or microsphere is retained or secured within the through hole by an interference or friction fit.

An opening to the through hole is preferably circular.

The through hole has a circular cross-sectional shape or profile. According to an embodiment the through holes or recesses may have a circular cross-section along at least 5%, 10%, 15%, 20%, 25%, 30%, 35%, 40%, 45%, 50%, 55%, 60%, 65%, 70%, 75%, 80%, 85%, 90%, 95% or 100% of the length or depth of the through hole.

The diameter of the through hole is preferably selected from the group consisting of: (i) < 0.5 mm; (ii) 0.5-1.0 mm; (iii) 1.0-1.5 mm; (iv) 1.5-2.0 mm; (v) 2,0-2.5 mm; (vi) 2.5-3.0 mm; (vii) 3.0-3.5 mm; (viii) 3.5-4.0 mm; (ix) 4.0-4,5 mm; (x) 4.5-5.0 mm; (xi) < 5.0 mm; and (xii) > 5.0 mm.

The depth of the through hole is preferably selected from the group consisting of: (i) < 0.5 mm; (ii) 0.5-1.0 mm; (iii) 1.0-1.5 mm; (iv) 1.5-2,0 mm; (v) 2.0-2.5 mm; (vi) 2.5-3.0 mm; (vii) 3.0-3.5 mm; (viii) 3.5-4.0 mm; (ix) 4.0-4.5 mm; (x) 4.5-5.0 mm; (xi) < 5.0 mm; and (xii) > 5.0 mm.

According to an embodiment in at least one sample well (or in all the sample wells) the base portion preferably comprises a plurality of open through holes wherein at least some (or all) of the plurality of open through holes are arranged so that there is no direct line of sight between reagent beads retained or secured in adjacent open through holes.

In at least one sample well (or in all the sample wells) the base portion may comprise a plurality of open through holes and wherein the base portion is segmented into a plurality of segments which are arranged at different heights relative to each other.

In at least one sample well (or in all the sample wells) the base portion may comprise a plurality of open through holes and wherein the sample well further comprises one or more baffles or dividers which preferably separates or divides the base portion into at least a first region and a second region.

The one or more baffles or dividers are preferably arranged so as: (i) to attenuate or eliminate light reflected off one or more reagent beads located In the first region from impinging upon one or more reagent beads located in the second region; and/or (ii) to attenuate or eliminate light reflected off one or more reagent beads located in the second region from impinging upon one or more reagent beads located in the region.

One or more open through holes may comprise a countersunk or enlarged portion for facilitating the insertion of a reagent bead or microsphere into one or more of the through holes.

The one or more sample wells preferably comprise at least 1, 2, 3, 4, 5, 6, 7, 8, 9, 10, 11, 12, 13, 14, 15, 16, 17, 18, 19, 20 or 21 through holes which are each arranged and adapted to receive, in use, a reagent bead or microsphere.

The one or more through holes provided in the base portion are preferably arranged:
(i) circumferentially around a central portion of the sample well; or
(ii) with a plurality of through holes arranged circumferentially around a central through hole; or
(iii) in a substantially close-packed manner; or
(iv) in a substantially symmetrical or asymmetrical manner; or
(v) in a substantially linear or curved manner; or
(vi) in a substantially regular or irregular manner; or
(vii) in an array; or
(viii) in a circle or two or more concentric circles with no through hole located at the centre of the base portion.

The sample plate preferably comprises sample wells arranged in an A x B format wherein;
A is selected from the group consisting of: (i) 1; (ii) 2; (iii) 3; (iv) 4; (v) 5; (vi) 6; (vii) 7; (viii) 8; (ix) 9; (x) 10; and (xi) > 10; and
B is selected from the group consisting of: (i) 1; (ii) 2; (iii) 3; (iv) 4; (v) 5; (vi) 6; (vii) 7; (viii) 8; (ix) 9; (x) 10; and (xi) > 10.

One or more of the sample wells are preferably interconnected to one or more other sample wells by one or more frangible regions or connections so that the sample plate can be separated by a user into a plurality of smaller sample plates, sample strips or individual sample wells.

The sample plate preferably comprises an immunoassay sample plate.

The sample plate may comprise a hybridization probe for detecting the presence of complementary DNA or RNA samples.

The sample plate may comprise a base having a female, male or other docking portion for securing the sample plate to a corresponding male, female or other docking portion of a plate frame holder.

According to an aspect of the present invention there is provided a combination of a sample plate as described above and one or more reagent beads or microspheres inserted or located in one or more of the through holes of the one or more sample wells.

At least some or substantially all of the reagent beads or microspheres preferably carry, comprise or are otherwise coated with a reagent, wherein the reagent is arranged and adapted to assay for an analyte of interest in a sample liquid.

At least some or substantially all of the reagent beads or microspheres preferably carry, comprise or are otherwise coated with a nucleic acid probe, wherein the nucleic acid probe is arranged and adapted to hybridize with single-stranded nucleic acid, DNA or RNA.

According to an embodiment there is provided a combination of a plate frame holder and a sample plate as described above.

The plate frame holder preferably comprises a male, female or other docking portion for firmly securing the sample plate to the plate frame holder.

According to an embodiment there is provided an automated apparatus comprising:
one or more reagent bead or microsphere dispensers;
a sample plate as described above; and
a control system arranged and adapted to control the dispensing of reagent beads or microspheres from the one or more reagent bead or microsphere dispensers into one or more sample wells of the sample plate.

The one or more reagent bead or microsphere dispensers preferably comprise:
a syringe body comprising an annular chamber surrounding a longitudinal bore, wherein the annular chamber Is arranged, in use, to channel or funnel reagent beads or microspheres provided within the annular chamber towards a chamber provided in the bore;
a plunger provided within the longitudinal bore; and
a barrel or nozzle;
wherein the plunger is arranged, in use, to dispense a reagent bead or microsphere from the chamber into the barrel or nozzle.

According to an embodiment there is provided an apparatus for assaying a liquid for one or more analytes of interest, the apparatus comprising:
one or more reagent bead or microsphere dispensers; and
a sample plate as described above.

According to an aspect of the present invention there is provided a method as claimed in claim 6.

According to an embodiment there is provided a method of using a sample plate to analyse a sample for multiple analytes comprising:
providing a sample plate as described above;
inserting one or more reagent beads or microspheres into one or more through holes or recesses of a sample well; and
adding a sample to the sample well.

The reagent beads or microspheres may be inserted into one or more of the pockets, recesses or bores of the sample wells either by the sample plate manufacturer of by the end user.

According to an aspect of the present invention there is provided a method of using an Enzyme Linked ImmunoSorbent Assay (ELISA) to detect an antigen or an antibody in a sample comprising:
providing a sample plate as described above;
inserting one or more reagent beads or microspheres into one or more through holes or recesses of a sample well; and
adding a sample to the sample well.

The reagent beads or microspheres may be inserted into one or more of the pockets, recesses or bores of the sample wells either by the sample plate manufacturer of by the end user.

According to an aspect of the present invention there is provided a method of using a nucleic acid probe to detect a DNA or RNA sequence in a sample comprising:
providing a sample plate as described above;
inserting one or more reagent beads or microspheres into one or more through holes or recesses of a sample well; and
adding a sample to the sample well.

The reagent beads or microspheres may be inserted into one or more of the pockets, recesses or bores of the sample wells either by the sample plate manufacturer of by the end user.

The plurality of beads preferably comprise one or more probes.

The probe preferably comprises a nucleic acid, antibody, antibody fragment, protein, peptide, aptamer, or chemical compound.

According to an embodiment the probe is an oligonucleotide.

According to the preferred embodiment the reagent beads are preferably opaque and signal is preferably only taken from the top of the bead. The bottom of the bead below a press fit or interference fit line does not, preferably, come into contact with sample fluid. In the preferred embodiment, in use, a reagent bead preferably forms a substantially fluid-tight seal with the cylindrical or section of the bore. A sample plate with inserted reagent beads according to the preferred embodiment therefore resembles fairly closely an empty conventional sample well.

According to an embodiment the reagent beads may protrude above the bottom of the sample well avoiding forming a moat region around the upper portion of the bead which could trap fluid.

However, according to other embodiments the reagent beads may be arranged so as not to protrude above the bottom of the sample well in which case they are also preferably protected and are not susceptible to damage through handling, pipetting or washing.

Beads are pressed or inserted into the bores formed in the base portion of the sample wells. The tops of the reagent beads once inserted either protrude above the bottom of the sample well according to the preferred embodiment or alternatively, according to less preferred embodiments, may be flush or level with the bottom of the sample well.

According to the preferred embodiment a 2 mm bead may be arranged to protrude 0.5 mm above the bottom of the base portion of the sample well. According to embodiments of the present invention one or more of the reagent beads may be arranged to protrude a distance of 0-5%, 5-10%, 10-15%, 15-20%, 20-25%, 25-30%, 30-35%, 35-40% or > 40% of the diameter of the bead above the bottom of the base portion of the sample well.

According to the preferred embodiment the depth of the bore is preferably selected from the group consisting of: (i) < 0.5 mm; (ii) 0.5-1.0 mm; (iii) 1.0-1.5 mm; (iv) 1.5-2.0 mm; (v) 2.0-2.5 mm; (vi) 2.5-3.0 mm; (vii) 3.0-3.5 mm; (viii) 3.5-4.0 mm; (ix) 4.0-4.5 mm; (x) 4.5-5.0 mm; (xi) < 5.0 mm; and (xii) > 5.0 mm.

An advantageous aspect of the disclosed embodiments is that since the reagent beads may be arranged to be inserted so that they are flush with the bottom of the well then the sample plate can be used with known automated microplate processing systems requiring only minimal hardware modifications. Furthermore, the sample well according to such an embodiment is essentially a cylinder having proportions which are similar to that of a well of a conventional microplate so the fluid and other handling characteristics of the sample well are well know. Processing steps according to such an embodiment such as pipetting, mixing, washing and incubation preferably follow the same type of fluid characteristics that conventional microplates go through.

The sample plate according to the preferred embodiment preferably has a fluid capacity of approximately 600 µl but advantageously, in use, only a small fraction of the total fluid capacity of a sample well is required in order to cover all the reagent beads disposed in the base of the sample plate.

Another advantageous feature of the sample plate according to the preferred embodiment is that fluid can be dispensed directly into the centre or central region of a sample well and according to the preferred embodiment the sample plate may be arranged so that no bores for securing reagent beads are arranged in the central region of the sample well. Such an arrangement is particularly advantageous in that reagent which preferably coats the reagent beads is not inadvertently washed off the reagent beads by the force of the fluid jet from awash head or pipette tip.

The sample plate according to the preferred embodiment preferably enables multiple tests to be carried out in a single sample well. This is achieved by inserting different reagent beads into separate bores in the same sample well thereby enabling multiplexing to be performed. According to the preferred embodiment reagent beads can be pressed into non-tapered holes in the base of the well as desired which results in a high degree of flexibility and the ability to use the entire sample well with a high efficiency.

A sample plate according to an embodiment of the present invention may comprise one or more 12 mm diameter sample wells. Each sample well may have a cross sectional surface area of 58 mm² and in total 54 sample wells of this size can be fitted into a conventional microplate footprint. Within each sample well a varied number of beads can be inserted. The bores in a sample well can have different diameters to accommodate different size reagent beads if desired.

According to other embodiments one or more sample wells may comprise 6 x 3.0 mm diameter pockets, recesses or bores, 10 x 2.0 mm diameter pockets, recesses or bores or 21 x 1.75 mm pockets, recesses or bores. The central region of the sample well is preferably kept free of pockets, recesses or bores. The bores may be arranged in a circle or two or more concentric circles or other patterns about the central region of the sample well.

According to an embodiment a sample plate having an array of 9 x 6 sample wells may be provided. If six bores are provided per sample well, then the sample plate can accommodate 324 reagent beads per plate. If 10 bores are provided per sample well, then the sample plate can accommodate 540 reagent beads per plate. If 21 bores are provided per sample well, then the sample plate can accommodate 1134 reagent beads per plate.

A further advantageous aspect of the present invention is that the sample plate according to the present invention is relatively simple to manufacture compared with other known arrangements. The sample plate can be manufactured by moulding using an open and shut tool so that the manufacturability is high and reliable. The injection mould tool design used to formed the sample plates is simple and does not require the use of undercuts or thin features to mould. As a result, the production of sample plates having different formats can be readily achieved. A tool that produces a sample well with six pockets or bores can be readily adapted to produce a sample well having a different number (e.g. 21) of pockets.

Another advantage of the preferred embodiment is that validation of different well designs and formats can be achieved simply since the test protocols can remain essentially the same. Pipetting and incubation do not change and the washing procedure would only requires, at most, a minor alteration to the aspirate routine.

It is apparent, therefore, that the sample plate according to the present invention is particularly advantageous compared to other known sample plates such as the sample plate disclosed in US-5620853.

The one or more bores may comprise a countersunk or enlarged portion for facilitating the insertion of a reagent bead or microsphere into one or more of the bores.

The one or more bores provided in the base portion are preferably arranged: (i) circumferentially around a central portion of the sample well; and/or; and/or (ii) in a substantially close-packed manner; and/or (iii) in a substantially symmetrical or asymmetrical manner; and/or (iv) in a substantially linear or curved manner; and/or (v) in a substantially regular or irregular manner; and/or (vi) in an array; and/or (vii) in a circle or two or more concentric circles with no bore located at the centre of the base portion.

The sample plate is preferably fabricated or otherwise made from polystyrene.

The sample plate may comprise either a strip or an array format. For example, according to a preferred embodiment the sample plate may comprise a 6x1 strip of sample wells. According to another preferred embodiment the sample plate may comprise nine 6x1 sample strips of sample wells.

According to an embodiment one or more of the sample wells may be interconnected to one or more other sample wells by one or more frangible regions or connections so that the sample plate can be separated by a user into a plurality of smaller sample plates, sample strips or individual sample wells. This enables a sample plate to be snapped or broken into a plurality of smaller sample plates. For example, a 6x1 strip of sample wells may be snapped into six individual sample wells or into two 3x1 sample strips.

According to the preferred embodiment individual sample wells, sample strips and sample plates are made from polypropylene. Sample wells, sample strips and sample plates are preferably made from a non-binding material such as polypropylene to ensure non-specific binding in the well is kept to a minimum.

A plate frame arranged to hold a plurality of sample wells, sample strips or one or more sample plates is preferably made from a plastic such as Acrylonitrile Butadiene Styrene ("ABS"). The plate frame is preferably made from a material which provides high rigidity and which ensures that sample wells, sample strips or one or more sample plates are held securely in place and remain flat after sample wells, sample strips or sample plates are secured into the plate frame. The plate frame is sufficiently robust to withstand handling by a user.

One or more of the sample wells may be interconnected to one or more other sample wells by one or more frangible regions or connections so that the sample plate can be separated by a user into a plurality of smaller sample plates, sample strips or individual sample wells.

At least some or substantially all of the reagent beads or microspheres which are dispensed, in use, into one or more of the bores carry or comprise a reagent, wherein the reagent is arranged and adapted: (i) to analyse samples; and/or (ii) to analyse samples by nucleic acid amplification reactions; and/or (iii) to analyse samples by polymerase chain reactions (PCR); and/or (iv) to analyse samples by an immunoassay process; and/or (v) to analyse samples by using a hybridization probe technique.

At least some or substantially all of the reagent beads or microspheres which are dispensed, in use, into one or more of the bores comprise polystyrene, plastic or a polymer.

The sample plate disclosed herein can comprise one or more beads. The bead can be a microparticle, particle, microsphere, or grammatical equivalents. The bead composition is dependent on the type of assay being performed. The bead may be composed of plastics, ceramics, glass, polystyrene, methylstyrene, acrylic polymers, paramagnetic materials, thoria sol, carbon graphite, titanium dioxide, latex or cross-linked dextrans such as Sepharose, cellulose, nylon, cross-linked micelles, Teflon or any combination thereof. In one embodiment, a bead comprises polystyrene, plastic, a polymer, or a combination thereof. In another embodiment, a bead comprises a ferrous or magnetic coating or has a ferrous or magnetic property. In yet another embodiment, a bead comprises an anti-static coating or has an anti-static property. The bead used in the sample plate reagent beads can be translucent, slightly translucent, or opaque. Commercially available beads can also be used.

The beads may be porous. The bead size may range from nanometers to millimeters. The bead may have a diameter of at least 0.1 mm. The bead may have a diameter of between 0.1 mm and 10 mm. In one embodiment, the bead may have a diameter of greater than about 0.5 mm; 0.5-1.0 mm; 1.0-1.5 mm; 1.5-2.0 mm; 2.0-2.5 mm; 2.5-3.0 mm; 3.0-3.5 mm; 3.5-4.0 mm; 4.0-4.5 mm; 4.6-5.0 mm; or greater than about 5.0 mm. The bead has a diameter greater than the diameter of a bore of a sample well.

A bead within the sample plate may comprise a reagent or probe, or be coated with a reagent or probe. The reagent or probe can be used to analyze a sample, such as by detecting an analyte. The probe or reagent can be attached to the bead. The attachment can be a covalent or non-covalent interaction. The probe can be a nucleic acid, antibody, antibody fragment, protein, peptide, aptamer, or chemical compound. For example, the probe can be an oligonucleotide. In one embodiment, the probe can be used to detect an analyte in a biological sample. In yet another embodiment, the probe can be used to for drug screening. For example, a library of compounds or antibodies can be screened for its binding ability to a protein or nucleic acid probe.

The probe can be used to provide detect a biomarker for a diagnosis or prognosis of a disease or condition, drug response or potential drug response, or for monitoring the progression of a disease or condition. For example, the probe can be an antibody or fragment thereof that is used to detect an antigen that is a biomarker for cancer. In another embodiment, the probe can be an antigen, peptide or protein, which is used to detect an antibody in a sample, which can be an indicative of a disease or condition.

The sample plate disclosed herein can comprise a plurality of probes, wherein a subset of the plurality differs from another subset of the plurality. The plurality of probes can be attached to beads. The different probes can be used to detect different analytes, thus allowing multiplexing with the sample plates disclosed herein. The sample plate can comprise at least 2, 3, 4, 5, 6, 7, 8, 9, 10, 11, 12, 13, 14, 15, 16, 17, 18, 19, or 20 different probes. The probes can be of the same type (for example, different antibodies) or of a different type (for example, a combination of nucleic acid probe(s) and antigen(s)).

According to an embodiment the one or more of the reagent bead or microsphere dispensers preferably comprise a tube containing, in use, a plurality of reagent beads or microspheres.

The apparatus preferably further comprises one or more sensors for sensing whether or not one or more reagents beads have been dispensed from one or more of the reagent bead or microsphere dispensers.

The apparatus preferably further comprises a translation stage for moving the sample plate relative to one or more reagent bead or microsphere dispensers.

The control system is preferably arranged and adapted to control the translation stage so that one or more reagent beads or microspheres from a reagent bead or microsphere dispenser are dispensed sequentially into different reagent bead or microsphere receiving chambers by moving the sample plate relative to the reagent bead or microsphere dispenser.

According to an embodiment the apparatus further comprises a fluid dispensing device for dispensing fluid into the sample wells of a sample plate.

The fluid dispensing device is preferably arranged and adapted to dispense x ml of fluid at a time into the one or more fluid receiving areas of one or more sample wells, wherein x is preferably selected from the group consisting of: (i) < 10; (ii) 10-20; (iii) 20-30; (iv) 30-40; (v) 40-50; (vi) 50-60; (vii) 60-70; (viii) 70-80; (ix) 80-90; (x) 90-100; (xi) 100-110; (xii) 110-120; (xiii) 120-130; (xiv) 130-140; (xv) 140-150; (xvi) 150-160; (xvii) 160-170; (xviii) 170-180; (xix) 180-190; (xx) 190-200; and (xxi) > 200.

According to an embodiment the apparatus further comprises an image analysis device or camera for determining whether or not a reagent bead or microsphere has been dispensed or is otherwise present in a bore of the sample plate.

The sample plate preferably has a first colour (or is transparent) and the reagent beads or microspheres preferably have a second different colour which contrasts with the first colour (or transparency) in order to facilitate visual detection of the presence or absence of a reagent bead or microsphere in a bore of the sample plate.

According to an embodiment the sample plate may further comprise a luminescence or fluorescence marker.

The apparatus may further comprise a luminescence or fluorescence detecting device for determining whether or not a reagent bead or microsphere has been dispensed or is otherwise present in a bore of the sample plate by determining whether or not a reagent bead or microsphere obstructs or partially obstructs the luminescence or fluorescence marker.

The apparatus may further comprise a magnetic and/or electrical and/or capacitive and/or mechanical sensor for sensing whether or not a reagent bead or microsphere has been dispensed or is otherwise present in a bore of a sample plate.

The control system may determine the number of reagent beads or microspheres present and/or the number of reagent beads or microspheres absent and/or the number of reagent beads or microspheres dispensed and/or the number of reagent beads or microspheres desired to be dispensed in a sample well.

According to an embodiment the control system may measure and/or adjust the volume of fluid dispensed or desired to be dispensed into a sample well dependent upon the number of reagent beads or microspheres determined to be present and/or absent and/or dispensed and/or desired to be dispensed in the sample well.

The control system may be arranged and adapted to ensure that the upper surface of at least some or substantially all reagent beads or microspheres located in the bores of a sample well are at least partially or fully immersed by a fluid when the fluid is dispensed into the sample well.

The control system is preferably arranged and adapted to ensure that the height of fluid dispensed into a sample well remains substantially constant irrespective of the number of reagent beads or microspheres present, absent, dispensed or desired to be dispensed into the sample well.

### BRIEF DESCRIPTION OF THE DRAWINGS

Various embodiments of the present invention will now be described, by way of example only, and with reference to the accompanying drawings in which:
Fig. 1 shows a sample well of a sample plate according to an embodiment of the present invention;
Fig. 2A shows a plan view of a sample well of a sample plate according to an embodiment, Fig. 2B shows in greater detail the bottom of a sample well according to an embodiment and Fig. 2C shows a reagent bead or microsphere dispensed in a sample well according to an embodiment;
Fig. 3A shows a reagent bead or microsphere dispenser and Fig. 3B shows a cutaway view of the reagent bead or microsphere dispenser;
Fig. 4 shows an exploded view of the reagent bead or microsphere dispenser;
Fig. 5 shows a microarrayer comprising a reagent bead or microsphere syringe pick-up device mounted on an x-y-z translation stage and engaged with a reagent bead or microsphere dispenser above a sample plate;
Fig. 6 shows in greater detail a cutaway view of a reagent bead or microsphere syringe pick-up device attached to a reagent bead or microsphere dispenser;
Fig. 7A shows a reagent bead or microsphere dispenser being transported by a reagent bead or microsphere syringe pick-up device and Fig. 7B shows a reagent bead or microsphere in the process of being dispensed from a reagent bead or microsphere dispenser by a plunger mechanism which is actuated by the reagent bead or microsphere syringe pick-up device;
Fig. 8A shows a reagent bead or microsphere syringe in the process of being ejected from the reagent bead or microsphere syringe pick-up device and Fig. 8B shows the reagent bead or microsphere syringe having been ejected from the reagent bead or microsphere pick-up device;
Fig. 9A shows nine sample strips loaded into a plate frame, wherein each sample strip comprises a 6x1 array of sample wells and Fig. 9B shows a plate frame into which a sample plate or one or more sample strips may be loaded;
Fig. 10A shows in greater detail a sample strip comprising six sample wells and Fig. 10B shows a sample strip comprising six sample wells being loaded into a plate frame;
Fig. 11A shows a single well being loaded into a plate frame, Fig. 11B shows in greater detail two sample wells connected by a break apart feature, Fig. 11C shows a sample well having an end feature and Fig. 11D shows a sample well having an ID and orientation tab;
Fig. 12A shows the underneath of a strip of sample wells, Fig. 12B shows a female alignment and retaining feature which helps to align a sample strip or sample well with a plate frame and Fig. 12C shows a corresponding male alignment and retaining feature which is provided in the base of the plate frame;
Fig. 13 shows a cross-sectional view of a strip of sample wells and shows an arrangement wherein the sample wells have a plurality of tapered bores wherein the angle of the taper is 6.0°;
Fig. 14A shows a further arrangement wherein conical through holes are provided in the base portion of a sample plate and reagent beads are loaded from the rear of the sample plate and Fig. 14B shows a sample plate according to a preferred embodiment wherein the sample plate has a cylindrical non-tapered through hole such that reagent beads may be loaded or inserted from the top through the sample well and are secured within the through hole by an interference fit;
Fig. 15 shows a sample strip comprising six sample wells wherein reagent beads are fitted from the underneath of the sample plate;
Fig. 16 shows a cross sectional 3D view of an arrangement showing reagent beads located within a concave end portion of a through hole;
Fig. 17 shows an embodiment wherein the base portion of a sample well is segmented into five segments and each base portion segment is arranged at a different relative height so that there is no direct line of sight between reagent beads inserted into open through holes provided in each base portion segment;

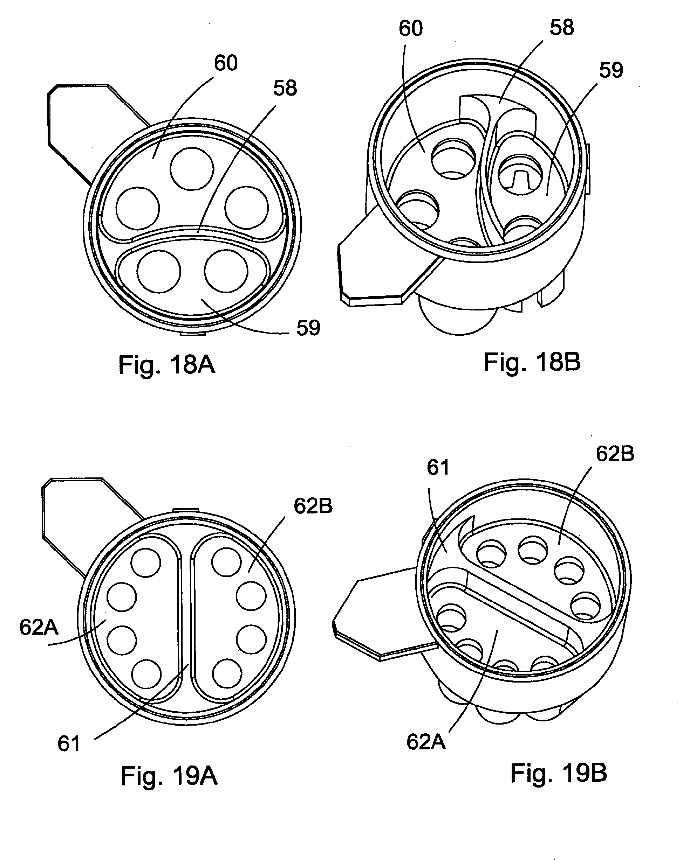

Fig. 18A shows a plan view of an embodiment wherein a relatively low-height baffle divides a base portion into two sections so that there is no direct line of sight between reagent beads inserted into open through holes provided in one section and reagent beads inserted into open through holes provided in the other section and Fig. 18B shows a 3D view of an embodiment wherein a low-height baffle separates the base portion into two sections so that there is no direct line of sight between reagent beads inserted into open through holes provided in one section and reagent beads inserted into open through holes provided in the other section; and
Fig. 19A shows a plan view of an embodiment wherein a relatively low-height baffle divides a base portion into two sections so that there is no direct line of sight between reagent beads inserted into open through holes provided in one section and reagent beads inserted into open through holes provided in the other section and Fig. 19B shows a 3D view of an embodiment wherein a relatively low-height baffle divides a base portion into two sections so that there is no direct line of sight between reagent beads inserted into open through holes provided in one section and reagent beads inserted into open through holes provided in the other section.

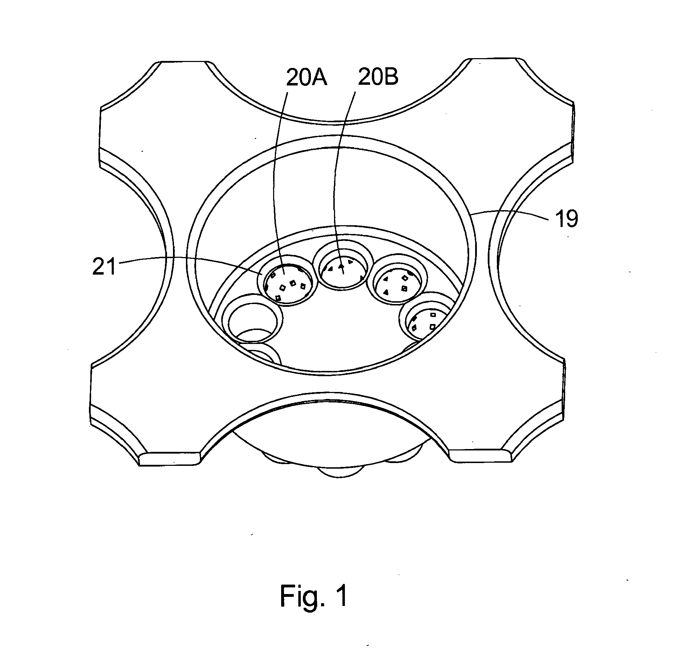

An embodiment of the present invention will now be described with reference to Fig. 1. A sample plate is provided which preferably comprises a plurality of sample wells 19 (although according to a less preferred embodiment a sample plate may be provided which comprises only a single sample well 19). According to a preferred embodiment the sample plate may comprise a 9x6 array of sample wells 19. A single sample well 19 is shown in Fig. 1 for ease of illustration. Embodiments are also contemplated wherein the sample plate may comprise a strip of sample wells 19 e.g. the sample plate may comprise, for example, a sample strip comprising an 1x9 or an 1x6 array of sample wells 19.

Each sample well 19 preferably comprises a plurality of bores 21 which are preferably provided in the base of the sample well 19. In the particular embodiment shown in Fig. 1 the sample well 19 comprises ten bores 21 which are formed or otherwise provided in the base of a sample well 19. Other embodiments are contemplated wherein a different number of bores 21 may be provided in the base of the sample well 19. For example, according to alternative embodiments at least some or all of the sample wells 19 provided in a sample plate may comprise 1, 2, 3, 4, 5, 6, 7, 8, 9, 11, 12, 13, 14, 15, 16, 17, 18, 19, 20, 21 or > 21 bores 21.

The bores 21 are preferably provided around the edge or perimeter of the sample well 19 and the centre or central region of the base of the sample well 19 is preferably substantially flat and free from bores 21,

According to an embodiment a plurality of reagent beads or microspheres each having a diameter of 1.75 or 2 mm may be loaded into a reagent bead or microsphere dispenser. According to another embodiment a reagent bead or microsphere dispensers may be provided which is arranged to handle reagent beads or microspheres having a diameter other than 1.75 mm or 2 mm. Less preferred embodiments are also contemplated wherein reagent beads or microspheres in a first reagent bead or microsphere dispenser may have a first diameter and wherein reagent beads or microspheres in a second different reagent bead or microsphere dispenser may have a second different diameter. Other less preferred embodiments are also contemplated wherein the reagent beads or microspheres loaded into a particular reagent bead or microsphere dispenser may have a plurality or mixture of different diameters.

The reagent beads or microspheres may be pre-loaded or pre-inserted into the bores 21 by a sample plate manufacturer. Alternatively, an end-user may load or insert the reagent beads or microspheres into the bores 21.

The reagent beads or microspheres preferably comprise a polystyrene, plastic or polymer core. The reagent beads or microspheres may be coated with a reagent (e.g. an antibody or antigen) which is preferably used to analyse samples. According to an embodiment the reagent may be used to analyse samples by polymerase chain reactions (PCR) or as part of an immunoassay procedure. Alternatively, according to an equally preferred embodiment the reagent may comprise a DNA or RNA sequence which is used as a hybridization probe to detect the presence of complementary DNA or RNA sequences in a sample. The reagent beads or microspheres may also be coated with an anti-static coating or may have an anti-static property.

A fluid to be tested is preferably dispensed into a sample well 19 of a sample plate. The fluid may, for example, comprise a sample of blood, serum, saliva or urine taken from a patient.

According to an embodiment 10-200 ml of fluid sample may be dispensed into each sample well 19 of a sample plate. According to the preferred embodiment less fluid may be dispensed into each sample well 19 compared with a conventional sample plate.

According to an embodiment a control system may be used to determine the location and/or type of reagent beads or microspheres which have been dispensed into the bores 21 of a sample well 19. Alternatively, the reagent beads or microspheres may be pre-loaded into the bores 21 of the sample wells 19. The control system may also determine into which bores 21 (if any) additional reagent beads or microspheres need to be dispensed. Once sample fluid has been dispensed into a sample well 19, the control system may check that an appropriate amount of sample fluid has been dispensed and that all the reagent beads or microspheres are at least partially or are fully immersed by the sample fluid.

The volume of sample fluid to be dispensed into a sample well 19 may depend upon the number of bores 21 formed within a sample well 19, the diameter of the reagent beads or microspheres which are dispensed or pre-loaded into the bores 21 and the extent to which reagent beads or microspheres protrude into the bottom of the sample well 19. The control system may be used to vary the amount of sample fluid dispensed into a sample well 19 so that reagent beads or microspheres are immersed in sample fluid to a substantially constant depth irrespective of the number of bores present in a sample well 19, the diameter of the reagent beads or microspheres or the extent to which the reagent beads or microspheres protrude into the base of the sample well 19.

Different formats of sample plates may be provided. For example, a sample plate may comprise a two dimensional array of sample wells 19 e.g. the sample plate may comprise a 4x4, 4x6, 4x8, 4x10, 4x12, 6x6, 6x8, 6x10, 6x12, 8x8, 8x10, 8x12, 10x10, 10x12 or 12x12 array of sample wells 19. According to other embodiments the sample plate may comprise a single dimensional strip of sample wells 19 e.g. the sample plate may comprise a 4x1, 6x1, 8x1, 10x1 or 12x1 strip of sample wells 19. Yet further embodiments are contemplated wherein the sample wells 19 may be provided in a format other than in an array or strip.

The base of the sample well 19 surrounding the bore may be arranged to have a countersunk portion in order to facilitate the insertion of a reagent bead or microsphere 20A;20B into the pocket, recess or bore 21. According to an embodiment the outer diameter of the countersunk portion may be 2.25 mm.

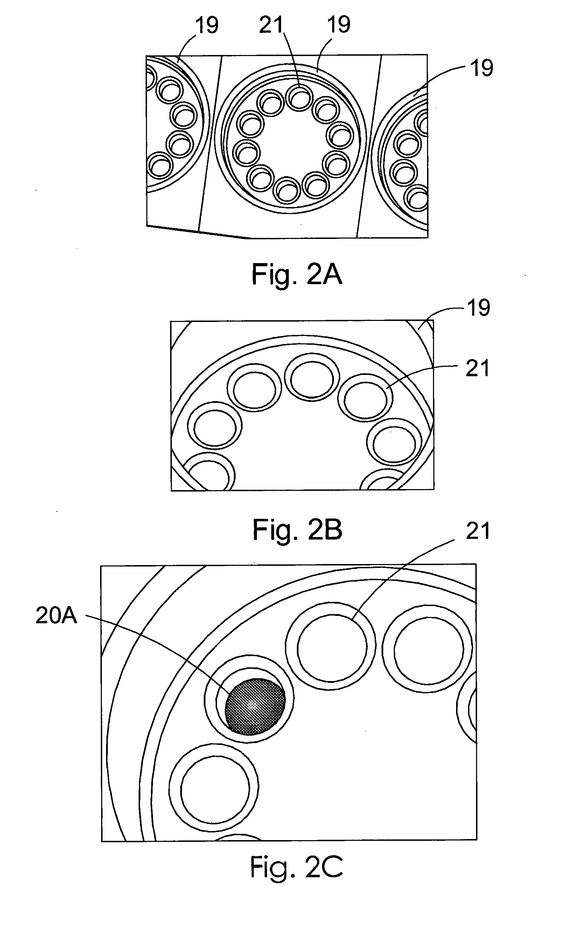

Fig. 2A shows a plan view of a sample well 19 and portions of two adjacent sample wells 19 which are provided in a sample plate. The sample wells shown in Fig. 2A form part of an array of sample wells 19 which are provided in the sample plate. Each of the sample wells 19 comprise ten bores 21 which are disposed in the bottom or base portion of the sample well 19. In use reagent beads or microspheres are preferably inserted into each of the bores 21 of a sample well 19 and with the embodiment shown in Figs. 2A-2C the reagent beads or microspheres are preferably secured in the bores 21.

Fig. 2B shows in greater detail the bottom of a sample well 19 and shows a plurality of bores 21 provided in the bottom portion of the sample well 19 each of which are arranged and adapted to receive a reagent bead or microsphere. Each of the bores 21 provided in the base of the sample well 19 preferably also comprises a countersunk portion or region at the entrance to each tapered bore. According to the preferred embodiment a single reagent bead or microsphere Is dispensed and inserted into each bore 21.

Fig. 2C shows in further detail a reagent bead or microsphere 20A disposed and securely located in a bore 21 provided In the base of a sample well 19. The reagent bead or microsphere 20A is secured within the bore 21. According to the embodiment shown in Fig. 2C the upper surface of the reagent bead or microsphere 20A when secured or located within the bore 21 is positioned or located approximately 0.3 mm below the surface of the well bottom. Therefore, according to this embodiment reagent beads or microspheres 20A located and secured in the bores 21 provided in the bottom of a sample well 19 do not project above the entrance to or surface of the bore 21 and hence do not project above the bottom surface of the sample well 19. However, according to other embodiments one or more reagent beads or microspheres may be located in one or more bores 21 provided in the base of the sample well 19 and may be located in relatively shallow bores 21 or when the microsphere 20A is securely positioned within the bore 21 then the reagent bead or microsphere projects above the entrance into or surface of the bore 21 and hence projects above the bottom surface of the sample well 19. According to a preferred embodiment reagent beads or microspheres 20A may be arranged such that they protrude 20-40% of their diameter above the bottom surface of the sample well.

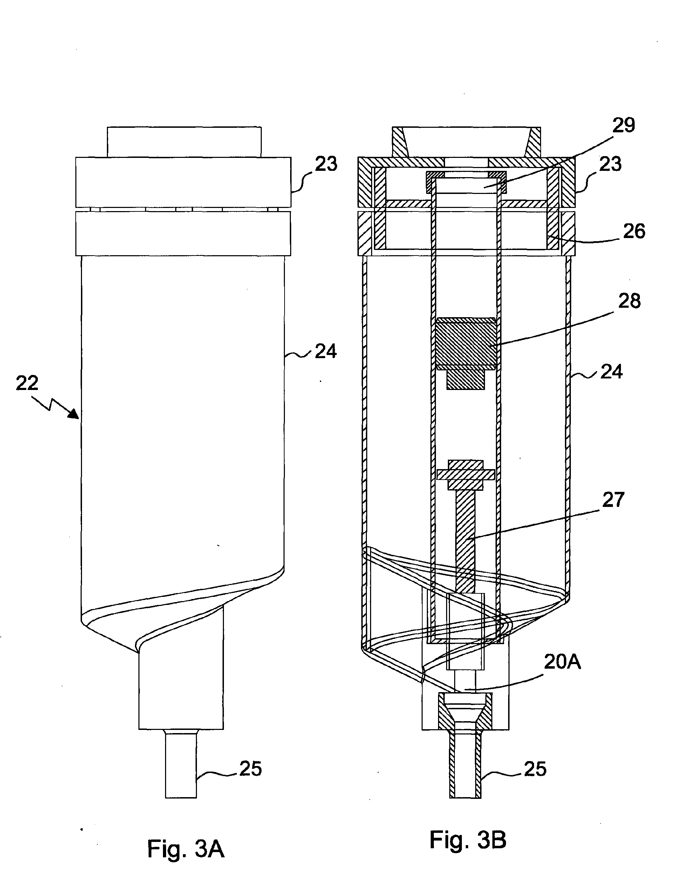

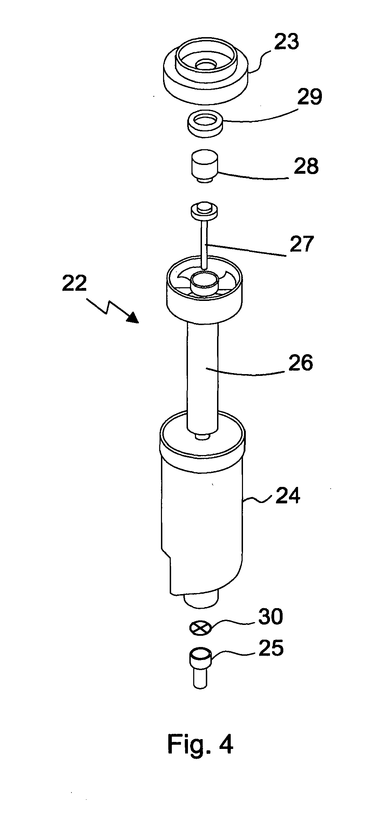

Reagent beads or microspheres may be dispensed into bores 21 provided in the bottom of a sample well 19 of a sample plate by means of a reagent bead or microsphere dispenser 22 as will now be described with reference to Figs. 3A, 3B and 4. The loading or dispensing of reagent beads or microspheres may be performed either by a sample plate manufacturer or by an end-user. A preferred reagent bead or microsphere dispenser 22 is shown in Fig. 3A and preferably comprises an upper cap 23, a syringe body 24 and a barrel 25 which projects from a lower region of the syringe body 24.

Fig. 3B shows a cutaway view of the reagent bead or microsphere dispenser 22 and shows that according to a preferred embodiment the reagent bead or microsphere dispenser further comprises a plunger guide 26 which is preferably positioned within the body of the syringe body 24. The plunger guide 26 preferably comprises a screw thread on the outer surface of an upper portion of the plunger guide 26. The inner surface of an upper portion of the syringe body 24 preferably comprises a complementary screw thread which engages with the screw thread provided on the outer surface of the upper portion of the plunger guide 26 so that in use the plunger guide 26 is secured or screwed firmly to the syringe body 24. The inner surface of the cap 23 also preferably comprises a screw thread and the cap 23 also preferably screws onto the upper portion of the plunger guide 26.

A plunger 27 is preferably located within the plunger guide 26 and the plunger 27 may be depressed by actuating an actuator or plunger boss 28 which is located above the plunger 27 in the bore defined by the plunger guide 26. An actuator spring (not shown) is provided between the actuator or plunger boss 28 so that when the actuator or plunger boss 28 is depressed, force is transmitted to the plunger 27 via the actuator spring causing the plunger 27 to become depressed. A return spring (not shown) is preferably provided between the bottom portion of the plunger guide 26 and the plunger 27 so that when the actuator or plunger boss 28 is no longer depressed, both the plunger 27 and the actuator or plunger boss 28 are preferably returned to an upper position.

Fig. 4 shows an exploded view of the reagent bead or microsphere dispenser 22 as shown and described above with reference to Figs. 3A and 3B. Fig. 4 also shows that a silicone member 30 is preferably provided within the upper portion of the barrel 25. In use, reagent beads or microspheres within the syringe body 24 are preferably funnelled or channelled by a helical path formed in the bottom section of the syringe body 24 so that at the bottom of the syringe body 24 reagent beads or microspheres become arranged in single file or in series. The single file or series of reagent beads or microspheres leads into a chamber which is preferably arranged immediately above the barrel 25 and below the plunger guide 26. The chamber is shaped and arranged so as to accommodate a single reagent bead or microsphere which is positioned in a bore below the plunger 27 and above the barrel 25. When the plunger 27 is depressed, the plunger 27 preferably pushes a single reagent bead or microsphere 20A located in the chamber in a downwards direction. The single reagent bead or microsphere 20A is preferably forced by the plunger 27 through the silicone member 30. According to the preferred embodiment the plunger 27 preferably continues to push or urge the reagent bead or microsphere 20A through the barrel 25 and into a bore 21 of a sample well 19 which is preferably positioned immediately below the barrel 25 of the reagent bead or microsphere dispenser 22. The silicone member 30 preferably prevents the accidental release of reagent beads or microspheres from the chamber of the reagent bead or microsphere dispenser 22 into the barrel 25 of the syringe body 24.

The bottom portion of the syringe body 24 preferably has a helical shape and acts to guide or channel reagent beads or microspheres towards the chamber disposed in a lower portion of the syringe body 24. The chamber is preferably arranged so that only a single reagent bead or microsphere sits above the silicone member 30 at any instance in time. The chamber is formed in the bore through which the plunger 27 travels and depression of the plunger 27 preferably causes a reagent bead or microsphere located in the chamber to be urged through the silicone member 30 and into the barrel 25.

A vibration mechanism may optionally be provided and may be arranged to act on the outside of the syringe body 24 so as to ensure that reagent beads or microspheres move down through syringe body 24 to the bottom portion of the syringe body 24 and line up in single file or in series ready to enter the chamber.

Reagent beads or microspheres may be pre-packed or pre-loaded into the syringe body 24 by, for example, a kit manufacturer or other supplier. Alternatively, an end-user may load the syringe body 24 with reagent beads or microspheres. According to another embodiment the sample plate manufacturer may load the syringe body 24 with reagent beads or microspheres and may supply sample plates, sample strips or individual sample wells which are pre-loaded with one or more reagent beads or microspheres.

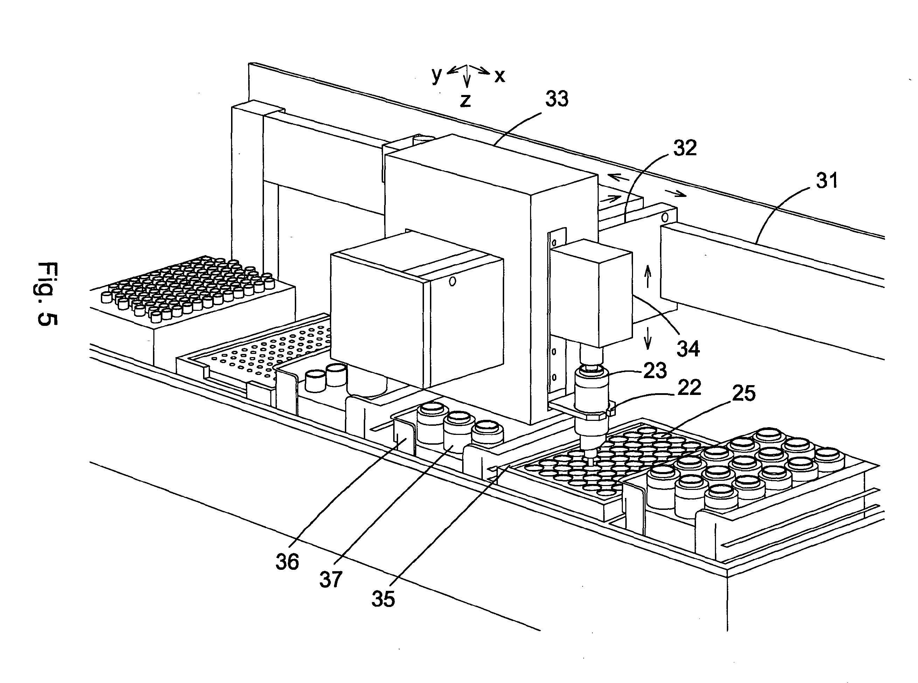

A microarrayer or automated apparatus will now be described with reference to Fig. 5. As shown in Fig. 5, a plurality of syringe bodies 37 may be loaded onto a tray or pack 36 which is then preferably automatically loaded into the microarrayer or automated apparatus. The tray or pack 36 comprising a plurality of syringe bodies 37 may be moved by a three-axis translation mechanism or robotic arm to a reagent bead or microsphere dispensing work area of the microarrayer or automated apparatus.

The microarrayer or automated apparatus preferably comprises a three-axis translation mechanism which preferably comprises a first translation stage comprising a guide rail 31 along which a first arm 32 may be translated in a first (x) horizontal direction. A second translation stage is preferably provided and comprises a mounting block 33 which preferably encompasses or surrounds the first arm 32. The mounting block 33 may be translated in a second (y) horizontal direction (which is preferably orthogonal to the first (x) horizontal direction) and may be moved backwards and forwards along the first arm 32. A third translation stage is preferably provided and preferably comprises a body or syringe drive mechanism 34 which preferably houses a linear actuator (not shown). The body or syringe drive mechanism 34 is preferably slidably mounted on the mounting block 33 and may be raised and lowered in a vertical (z) direction.

The three-axis translation mechanism preferably further comprises a retractable arm 35 which preferably extends from the mounting block 33. The three-axis translation mechanism is preferably programmed to select and pick up a reagent bead or microsphere dispenser 22,37 from the tray or pack 36 comprising a plurality of reagent bead or microsphere dispensers 22,37. The body or syringe drive mechanism 34 comprises a tapered spigot which is resiliently mounted within a tubular housing. The spigot is arranged to engage with a tapered portion provided on the syringe cap 23 of the reagent bead or microsphere dispenser 22,37. When a reagent bead or microsphere dispenser 22,37 is positioned in the tray or pack 36 the spigot may be lowered onto the syringe cap 23 of a reagent bead or microsphere dispenser 22,37 thereby securing the reagent bead or microsphere dispenser 22,37 to the body or syringe drive mechanism 34 in a detachable manner. The body or syringe drive mechanism 34 and attached reagent bead or microsphere dispenser 22,37 may then be raised to a height such that the retractable arm 35 (which is initially retracted within the body of the mounting block 33) can then be extended. The reagent bead or microsphere dispenser 22,37 is then lowered by the body or syringe drive mechanism 34 so that the upper portion of the syringe body 24 is secured by the retractable arm 35. The retractable arm 35 preferably has an aperture having an internal diameter which is preferably smaller than the outermost diameter of a rim of the upper portion of the syringe body 24.

According to the preferred embodiment each reagent bead or microsphere dispenser 22,37 preferably comprises a plurality of identical reagent beads or microspheres. According to an embodiment up to 15 separate reagent bead or microsphere dispensers 22,37 may be loaded or provided in a single tray or pack 36 and each of the reagent bead or microsphere dispensers 22,37 may have a capacity of up to approximately 2000 reagent beads or microspheres.

According to the preferred embodiment the syringe drive mechanism 34 is arranged to pick a reagent bead or microsphere dispenser 22,37 out of the tray or pack 36 and will position and lower the barrel 25 of the reagent bead or microsphere dispenser 22,37 so that it is immediately above a desired reagent bead or microsphere pocket or recess 21 provided in a sample well 19 of a sample plate. The syringe drive mechanism 34 is then preferably actuated so that the actuator or plunger boss 28 of the reagent bead or microsphere dispenser 22,37 is depressed which in turn causes the plunger 27 to push a reagent bead or microsphere 20A from the chamber through the silicone member 30, through the barrel 25 and into the desired reagent bead or microsphere pocket or recess 21 of the sample well 19. The syringe drive mechanism 34 is preferably arranged to depress the actuator boss 28 and plunger 27 with a desired amount of force as opposed to moving the actuator or plunger boss 28 and plunger 27 to a certain vertical position. As a result, reagent beads or microspheres 20A are preferably pressed in tightly and consistently into the reagent bead or microsphere pockets or recesses 21 of a sample well 19 with a constant amount of force.

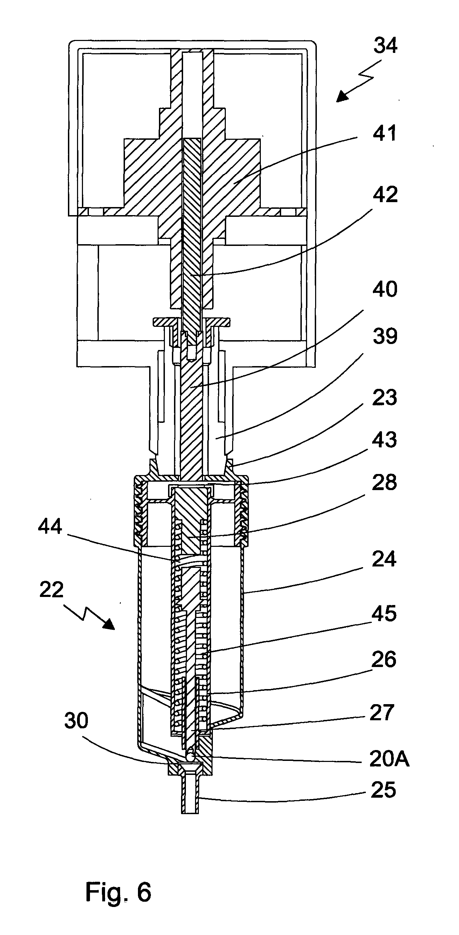

Fig. 6 shows in greater detail a reagent bead or microsphere dispenser pick-up device or syringe drive mechanism 34 during the process of picking up a reagent bead or microsphere dispenser 22. The reagent bead or microsphere dispenser pick-up device or syringe drive mechanism 34 comprises a spigot 39 having a tapered lower end which is arranged to engage with a tapered recess provided in the upper portion of the syringe cap 23 of the reagent bead or microsphere dispenser 22. The spigot 39 comprises a central bore through which a plunger push rod 40 is mounted. The plunger push rod 40 is arranged to be driven upwards or downwards by a linear actuator 41 which drives a linear actuator lead screw 42 which in turn raises or lowers the plunger push rod 40.

As shown in Fig. 6, in order to pick up a reagent bead or microsphere dispenser 22 the reagent bead or microsphere dispenser pick-up device or syringe drive mechanism 34 is lowered onto the reagent bead or microsphere dispenser 22 so that the spigot 39 of the reagent bead or microsphere pick-up device or syringe drive mechanism 34 engages with the syringe cap 23 of the reagent bead or microsphere dispenser 22. As the reagent bead or microsphere dispenser pick-up device or syringe drive mechanism 34 is driven downwards onto the reagent bead or microsphere dispenser 22, the spigot 39 becomes compressed and moves upwards until it is prevented from moving any further upwards. The spigot 39 is preferably driven further downwards whilst in a compressed state so that the interlocking tapers of the spigot 39 and syringe cap 23 preferably engage causing the reagent bead or microsphere dispenser 22 to become attached to the reagent bead or microsphere pick-up device or syringe drive mechanism 34.

The reagent bead or microsphere dispenser 22 as shown in Fig. 6 is substantially similar to that shown in Figs. 3A, 3B and 4 except that the spacer 29 shown in Figs. 3B and 4 is replaced with a retaining cap 43 in the embodiment shown in Fig. 6. Fig. 6 also shows the location of an actuating spring 44 which is provided between the actuator or plunger boss 28 and the plunger 27 and which transmits force applied to the actuator or plunger boss 28 to the plunger 27. A return spring 45 is also shown and is provided between the plunger 27 and the base of the plunger guide 26 and causes the plunger 27 (and hence also the actuator or plunger boss 28) to return to an upper position when the actuator or plunger boss 28 is no longer depressed or actuated.

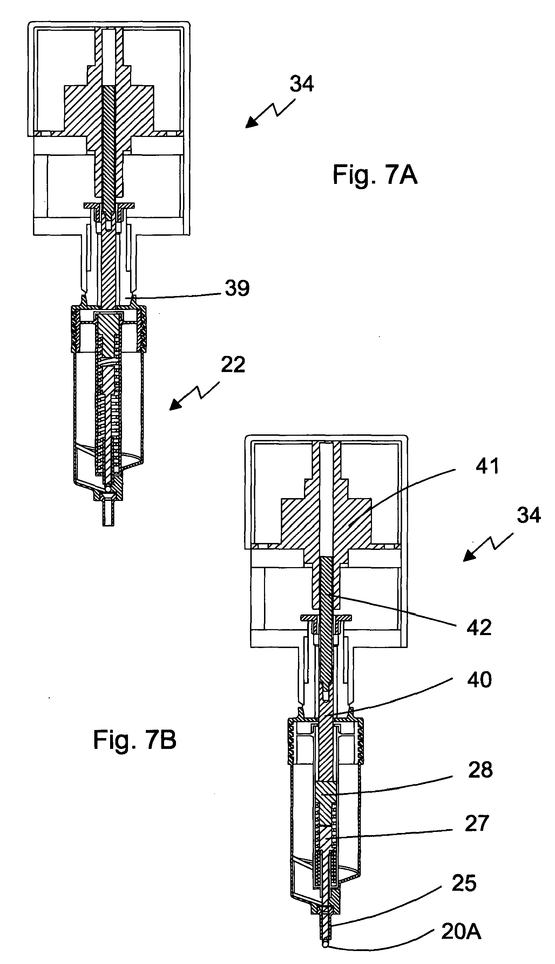

Fig. 7A shows the reagent bead or microsphere dispenser pick-up device or syringe drive mechanism 34 which has picked up a reagent bead or microsphere dispenser 22 and which is in the process of transporting the reagent bead or microsphere dispenser 22 to a desired location. Once the reagent bead or microsphere dispenser pick-up device or syringe drive mechanism 34 has engaged with the reagent bead or microsphere dispenser 22, the reagent bead or microsphere dispenser pick-up device or syringe drive mechanism 34 is raised so that the spigot 39 is no longer compressed. The spigot 39 returns to a downward position and the reagent bead or microsphere dispenser 22 including syringe body 24 is locked on to the spigot 39 by the tapers on the spigot 39 and syringe cap 23.

Fig. 7B shows a reagent bead or microsphere dispenser 22 in the process of dispensing a reagent bead or microsphere 20A from the reagent bead or microsphere dispenser 22 into a pocket of a sample well (not shown) of a sample plate (not shown). The linear actuator 41 of the reagent bead or microsphere dispenser pick-up device or syringe drive mechanism 34 is preferably actuated and causes the linear actuator lead screw 42 to extend thereby pushing the push rod 40 downwards. The downwards movement of the push rod 40 depresses the actuator or plunger boss 28. The actuator or plunger boss 28 transmits force to the plunger 27 via the actuating spring 44 and preferably does not touch the plunger 27 directly. The plunger 27 preferably forces a reagent bead or microsphere 20A from a chamber within the central bore provided within the syringe body 24. The reagent bead or microsphere 20A is preferably forced through the membrane 30 and down through the barrel 25 and into the pocket of a sample plate (not shown) by the plunger 27.

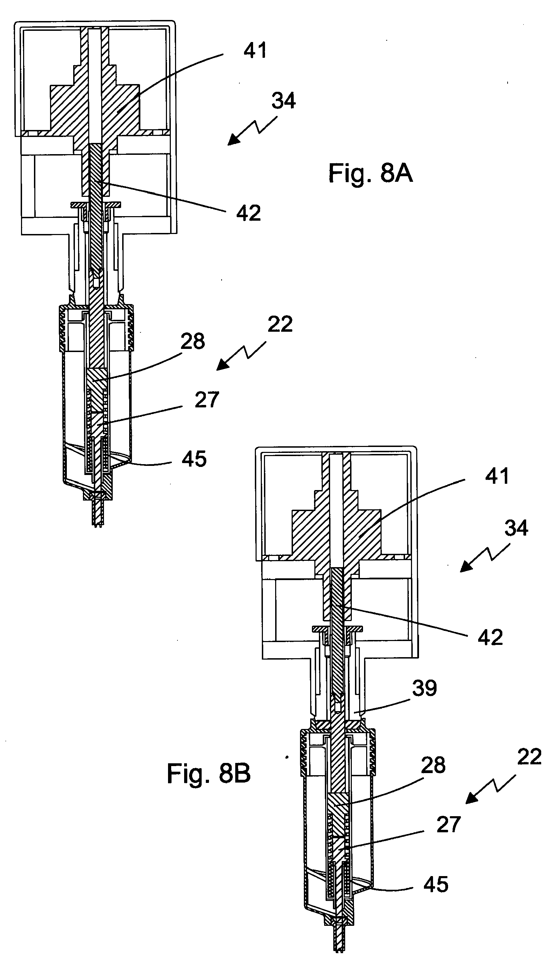

Fig. 8A shows the reagent bead or microsphere pick-up device or syringe drive mechanism 34 in the process of ejecting a reagent bead or microsphere dispenser 22 from the end of the reagent bead or microsphere pick-up device or syringe drive mechanism 34. In this mode of operation the reagent bead or microsphere dispenser 22 is positioned above the tray or pack 36. The linear actuator 41 preferably drives the linear actuator lead screw 42 downwards until the plunger 27 is extended a maximum extent. The spigot 39 is also extended to the maximum extent. The linear actuator 41 then preferably continues to apply force via the actuator or plunger boss 28 to the plunger 27, as shown in Fig. 8B, with the result that the body of the reagent bead or microsphere dispenser 22 is preferably forced off from the end of the tapered spigot 39. The reagent bead or microsphere dispenser 22 then preferably falls back into the reagent bead or microsphere dispenser tray or pack 36.

In order to illustrate aspects of an embodiment of the present invention a test was performed wherein a sample plate comprising nine sample wells 19 was provided. Each sample well 19 comprised ten bores 21 which were arranged in a circle around a central portion of the sample well 19. Each of the bores 21 were loaded with reagent beads or microspheres which were coated with different concentrations of reagent. The ten beads in the first sample well were coated with a reagent having a concentration of 10 µg/ml and the ten beads in the second sample well were coated with a reagent having a concentration of 8 µg/ml. The ten beads in the third sample well were coated with a reagent having a concentration of 4 µg/ml and the ten beads in the fourth sample well were coated with a reagent having a concentration of 2 µg/ml. The ten beads in the fifth sample well were coated with a reagent having a concentration of 1 µg/ml and the ten beads in the sixth sample well were coated with a reagent having a concentration of 0.5 µg/ml. The ten beads in the seventh sample well were not coated with a reagent i.e. the concentration was 0 µg/ml. The ten beads in the eighth sample well were coated with different concentrations of reagent and comprised concentrations of 10 µg/ml, 8 µg/ml, 4 µg/ml, 2 µg/ml, 1 µg/ml, 0.5 µg/ml, 0 µg/ml, 0 µg/ml, 0 µg/ml and 0 µg/ml. The ten beads in the ninth sample well had the same concentrations as the reagent beads or microspheres in the eighth sample well and were arranged in the same manner as the reagent beads or microspheres in the eighth sample well.

The reagent beads or microspheres were coated with a capture antibody comprising sheep IgG and were transported in a bicarbonate buffer containing 0.02% Kathon (RTM) preservative.

The sample wells 19 of the sample plate were emptied of the preservative in which the reagent beads or microspheres were transported in and 400 µl of a 1/1000 diluted donkey anti-sheep IgG peroxidise conjugate in a Tris Buffered Saline ("TBS") conjugate diluent buffer was added to each sample well 19. The sample plate was then incubated at ambient temperature and was subjected to medium intensity vibrations for a period of 45 minutes. Any unbound conjugate was then aspirated from the sample wells 19 using a single channel wash head of a microarrayer apparatus (DS2 (RTM), available from Dynex Technologies). Once any unbound conjugate had been aspirated from the sample wells 19, 500 µl of 1/20 diluted Tris Buffered Saline wash fluid was then immediately added to each sample well 19. The wash fluid was then aspirated from the sample wells 19 and the process of washing and aspirating wash fluid from the sample wells 19 was repeated twice more. After the third washing step including aspiration of wash fluid had been completed, 300 µl of luminol (a chemiluminescent marker) was then immediately added to each sample well 19. The sample plate was then incubated in the dark at ambient temperature whilst being subjected to medium intensity vibrations for 15 minutes. The sample plate was then transferred immediately to a reading chamber.

A camera was set to an exposure time of 6 minutes and 30 seconds with a gain of 20. Images were taken at 22 minutes and 29 minutes after luminol had been added. The camera exposure time was then changed to 8 minutes and 37 seconds. Further images were taken at 38 minutes, 47 minutes, 56 minutes and 65 minutes after luminol addition. Analysis of the images showed that the greatest observed signal strength was obtained after 15-22 minutes from luminol addition which is consistent with the luminol decay curve.

According to the preferred embodiment the following steps may be carried out once reagent beads or microspheres have been dispensed into bores of a sample plate. Firstly, sample fluid may be added to one or more sample wells of the sample plate. The sample fluid may comprise one or more analytes such as specific antigens which may react with reagent coated on one or more of the reagent beads or microspheres. The reagent beads or microspheres are preferably coated with a specific capture antibody.

Once the sample fluid has been added to the sample wells, the sample plate is then preferably subjected to an incubation step. After the sample plate has been subjected to an incubation step so that antigen-antibody complexes are formed, the sample plate is then preferably subjected to one or more washing and aspirate steps in order to remove any unbound sample fluid and to remove any wash fluid. An enzyme conjugate is then added which will bind to the antigen part of any antigen-antibody complexes which have been formed but which will not bind to antibodies or to the antibody part of an antigen-antibody complex. The sample plate is then incubated before being subjected to one or more washing and aspirate steps. Once the sample plate has been subjected to one or more washing and aspirate steps luminol (or another visualising agent) is preferably added. The sample plate is then preferably aspirated to remove any excess luminol (or other visualising agent). The luminol (or other visualising agent) upon contacting enzymes attached to the antigen part of an antigen-antibody complex will then breakdown causing a distinctive colour to be produced. In the final stage the sample plate is analysed and an endpoint determination is preferably made.

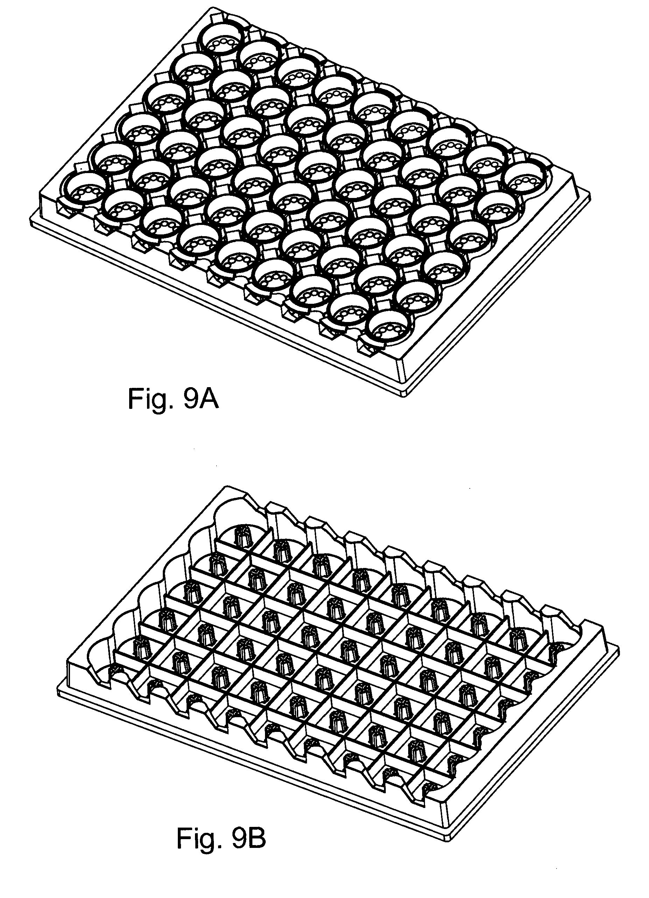

A particularly preferred embodiment of the present invention is shown in Figs. 9A and 9B and will be described in more detail below. Fig. 9A shows nine sample strips loaded into a plate frame. Each of the sample strips shown in Fig. 9A comprises a 6x1 strip of sample wells. The sample strips can be removeably loaded into the plate frame. Each of the nine sample strips comprises six sample wells and each sample well preferably comprises ten (optionally tapered) bores which, in use, are arranged to receive a reagent bead. The reagent beads are preferably loaded or pre-loaded into the bores such that the reagent beads protrude above the base portion of the sample well. Fig. 9B shows the plate frame into which the sample plates may be loaded in more detail.

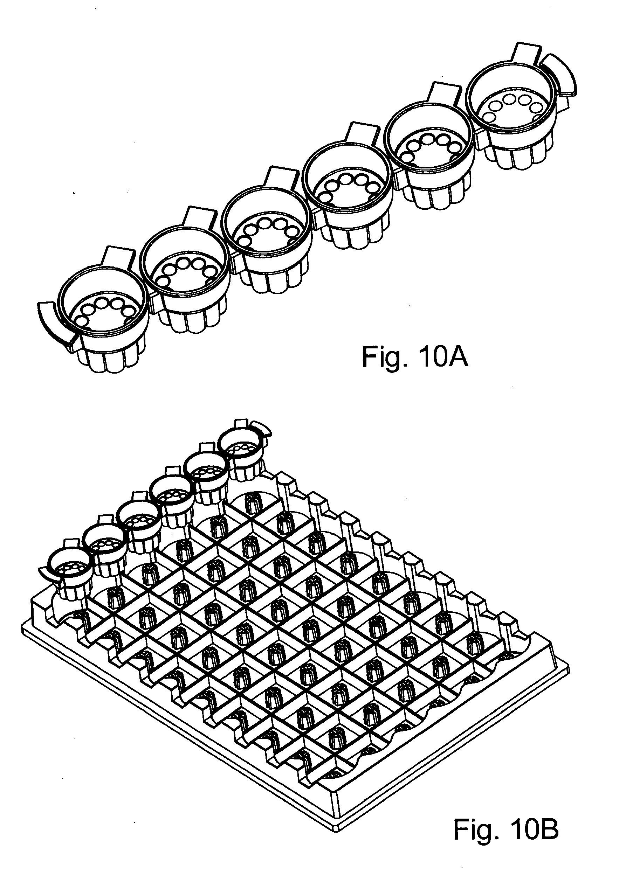

Fig. 10A shows in greater detail a sample strip comprising six sample wells. According to the preferred embodiment the sample wells in a strip can be separated or otherwise broken apart. According to an embodiment the sample plate or strip can be separated or divided up into single sample wells. Fig. 10B shows a sample strip comprising six sample wells being loaded into a plate frame.

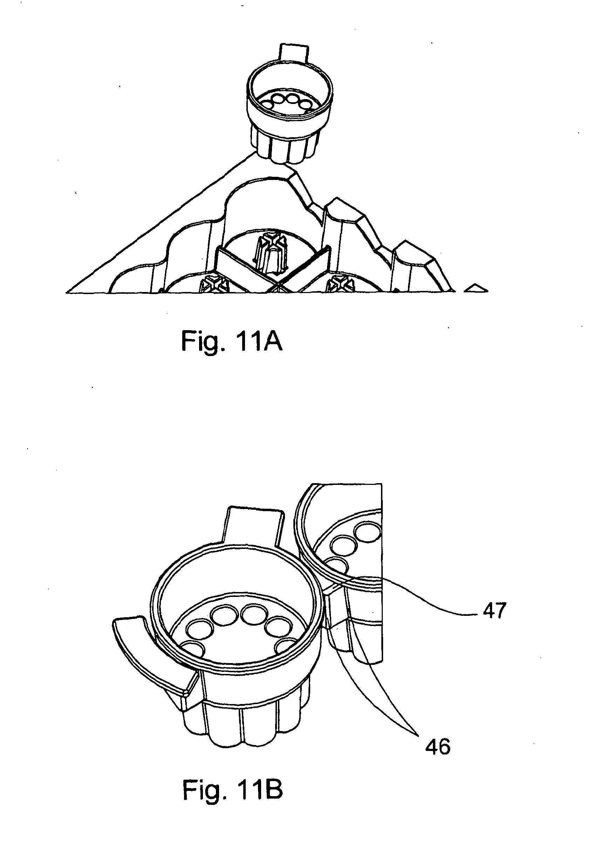

Fig. 11A shows a single sample well (which has been separated from a strip of sample wells) being loaded into a plate frame. The sample wells preferably comprise a female portion which is preferably arranged to engage or interlock with a male portion which is preferably provided on the base of the plate frame. The sample plate or sample strip is preferably arranged to be firmly secured and fixed to the plate frame when loaded onto the plate frame.

Fig. 11B shows in greater detail two sample wells which are connected by a break-apart feature 47. The break-apart feature 47 preferably allows a user to separate adjacent sample wells. According to an embodiment sample wells may be separated from each other but may still be placed next to each other on the plate frame without interfering with each other. The break-apart feature 47 preferably comprises one, two or more than two break points 46. According to an embodiment the connecting piece 47 between two sample wells may be separated from a sample well at a first break point 46. The connecting piece 47 may then be broken off or otherwise removed from the single sample well that it is attached to by breaking the connecting piece 47 from the sample well at a second break point 46.

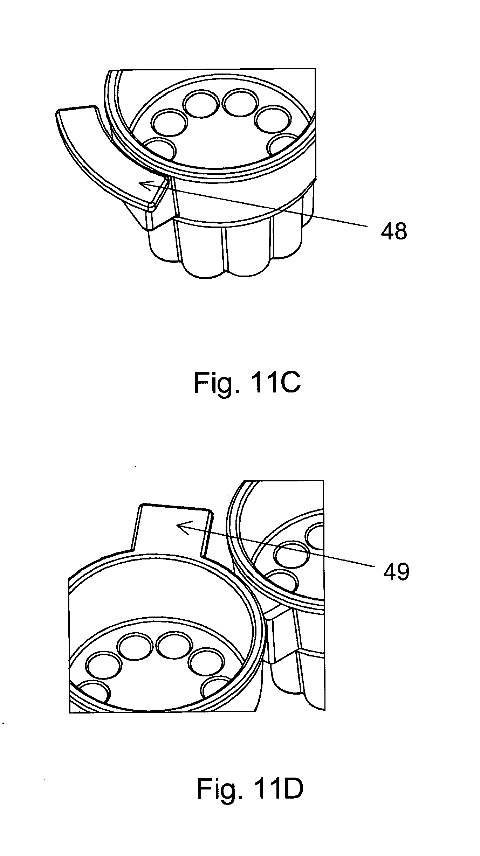

Fig. 11C shows a sample well having an end break-apart feature 48. The end break-apart feature 48 allows the end wells to be used singly in the plate frame without interfering with another sample well. The end break-apart feature 48 provides something for a user to hold in order to remove a strip of sample wells or a single sample well from the plate frame.

Fig. 11D shows a sample well having an ID and orientation tab 49. The tab 49 allows an identifier to be printed onto the tab 49 or to be otherwise attached to the tab 49. The identifier may comprise a 2D or 3D barcode and/or human readable text. The tab 49 preferably assists a user to orientate a sample well when a single sample well is used by aligning with features in the plate frame and/or on other sample wells.

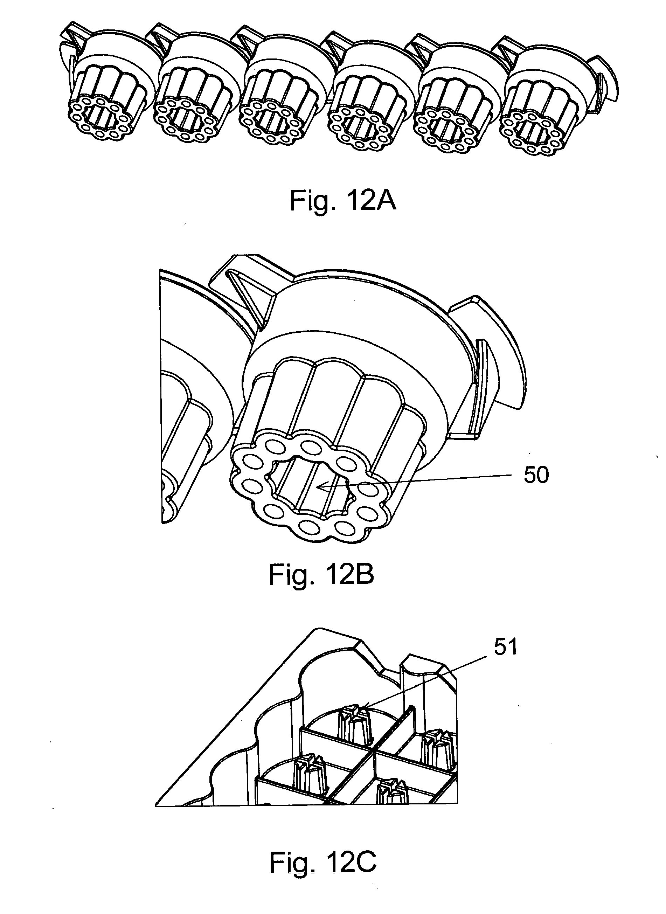

Fig. 12A shows the underneath of a strip of sample wells and shows that according to the preferred embodiment each sample well comprises ten bores or recesses in which a reagent bead is preferably inserted in use. The base or underside of each sample well preferably also comprises a female portion which is preferably arranged to be mated, in use, with a male portion which is provided in the base of the plate frame.

Fig. 12B shows in greater detail a female alignment and retaining feature 50 which helps to align a strip of sample wells with a plate frame. Fig. 12C shows a corresponding male alignment and retaining feature 51 which is preferably provided in the base of the plate frame. The male portion 51 may according to an embodiment comprise a plurality of flexible projections which are preferably deformed inwards as a sample well is located over the male portion 51. The projections on the plate frame preferably move or close together ensuring that the sample well is kept in place without having to apply undue force either to mount or fix a sample well onto the plate frame and/or to demount a sample well from the plate frame.

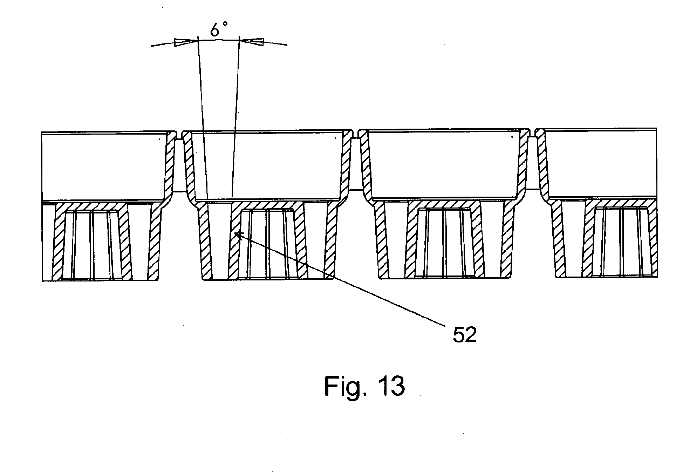

Fig. 13 shows a cross-sectional view of a strip of sample wells and shows that according to an arrangement the sample wells may comprise a plurality of tapered bores 52. The tapered bores 52 preferably act as pockets into which a reagent bead is inserted in use. The angle of the taper in the arrangement, shown in Fig. 13 is 6.0°.

Although various embodiments described above have focussed upon reagent beads which are coated with a biomolecule for use in an Immunoassay or ELISA procedure, the present invention equally applies to reagent beads which comprise or which are otherwise coated with a nucleic acid sequence and which are used as a hybridization probe for the detection of DNA or RNA sequences which are complementary to those provided on the reagent beads. As will be understood by those skilled in the art, the hybridization probe will be inactive until hybridization, at which point there is a conformational change and the molecule complex becomes active and will then fluoresce under UV light. Therefore, all the various embodiments described above and all the various aspects of the embodiments described above apply equally to the use of reagent beads comprising or which are otherwise coated with a DNA or RNA sequence (or other nucleotide sequence) for use as a hybridization probe to detect complementary DNA or RNA sequences.

Many variants, including fluorogenic and luminogenic substrates for ELISA, direct labeling of the second member of the binding pair with a fluorescent or luminescent molecule (in which case the procedure is not called an ELISA but the process steps are very similar) and nucleic acids or other specific pairing agents instead of antibodies can be used as a probe. The same principles can be used to detect or determine any materials which can form specific binding pairs, for example using lectins, rheumatoid factor, protein A or nucleic acids as one of the binding partners.

The sample plate can thus be used to detect an analyte, such as a biomarker, which can be indicative of a disease or condition. The disease or condition can be a tumor, neoplasm, or cancer, such as breast cancer, ovarian cancer, lung cancer, colon cancer, hyperplastic polyp, adenoma, colorectal cancer, high grade dysplasia, low grade dysplasia, prostatic hyperplasia, prostate cancer, melanoma, pancreatic cancer, brain cancer (such as a glioblastoma), hematological malignancy, hepatocellular carcinoma, cervical cancer, endometrial cancer, head and neck cancer, esophageal cancer, gastrointestinal stromal tumor (GIST), renal cell carcinoma (RCC) or gastric cancer. The disease or condition can also be an inflammatory disease, immune disease, or autoimmune disease, such as inflammatory bowel disease (IBD), Crohn's disease (CD), ulcerative colitis (UC), pelvic inflammation, vasculitis, psoriasis, diabetes, autoimmune hepatitis, Multiple Sclerosis, Myasthenia Gravis, Type I diabetes, Rheumatoid Arthritis, Psoriasis, Systemic Lupus Erythematosis (SLE), Hashimoto's Thyroiditis, Grave's disease, Ankylosing Spondylitis Sjogrens Disease, CREST syndrome, Scleroderma, Rheumatic Disease, organ rejection, Primary Sclerosing Cholangitis, or sepsis. The disease or condition can also be a cardiovascular disease, such as atherosclerosis, congestive heart failure, vulnerable plaque, stroke, ischemia, high blood pressure, stenosis, vessel occlusion or a thrombotic event. The disease or condition can also be a neurological disease, such as Multiple Sclerosis (MS), Parkinson's Disease (PD), Alzheimer's Disease (AD), schizophrenia, bipolar disorder, depression, autism, Prion Disease, Pick's disease, dementia, Huntington disease (HD), Down's syndrome, cerebrovascular disease, Rasmussen's encephalitis, viral meningitis, neurospsychiatric systemic lupus erythematosus (NPSLE), amyotrophic lateral sclerosis, Creutzfeldt-Jacob disease, Gerstmann-Straussler-Scheinker disease, transmissible spongiform encephalopathy, ischemic reperfusion damage (e.g. stroke), brain trauma, microbial infection, or chronic fatigue syndrome. The phenotype may also be a condition such as fibromyalgia, chronic neuropathic pain, or peripheral neuropathic pain. The disease or condition can also be an infectious disease, such as a bacterial, viral or yeast infection. For example, the disease or condition may be Whipple's Disease, Prion Disease, cirrhosis, methicillin-resistant staphylococcus aureus, HIV, hepatitis, syphilis, meningitis, malaria, tuberculosis, or influenza. Viral proteins, such as HIV or HCV-like particles can be assessed in an exosome, to characterize a viral condition.

The sample plate can be used to detect a biomarker that is used to detect the disease or condition. For example, the detection of a biomarker can be used to detect or provide a diagnosis, prognosis of a disease or condition. For example, the sample plate can comprise a probe for a cancer marker, and used to detect the cancer marker in a sample from an individual. The presence, absence, or level of the cancer marker in the sample can be indicative of cancer in the individual. In another embodiment, the sample plate can also be used to monitor a disease or condition. For example, an increased level of the cancer marker, as compared to a control, or compared to an earlier assay for the cancer marker from the same individual, can be indicative of progression of the cancer. In yet another embodiment, the sample plate can be used to in determine a therapy or course of action for a condition. For example, an individual may have a genetic variant which leads to the individual being unable to metabolize certain drugs. The sample plate can be used to detect the genetic variant. In another embodiment, the sample plate may be used to detect a compound, which can be indicative of a drug not being metabolized. The sample plate can also be used to detect the intake of certain drugs or compounds, such as be detecting the drug or by-products of the drug, which can be used for drug testing.

The sample plate can also be used to screen for drugs. For example, the sample plate can comprise a probe that is a target for drug development. The sample plate can then be used to screen a library of compounds. Alternatively, the sample plate can comprise a plurality of probes that comprise a library of compounds that are potential drugs. The sample can comprise a drug target, which is added to the sample plate.

Also provided herein is a kit comprising a sample plate disclosed herein. The kit can comprise one or more components for detecting an analyte or for performing an assay. A kit for detecting an analyte is disclosed and comprises one or more sample plates and a plurality of beads. The plurality of beads can comprise one or more probes, such as a probe that is a nucleic acid, antibody, antibody fragment, protein, peptide, aptamer, or chemical compound. A kit for performing an Enzyme Linked ImmunoSorbent Assay (ELISA) procedure is disclosed. The kit can comprise one or more sample plates as described herein; and a plurality of beads, wherein the beads are coated with a reagent comprising an antibody, an antigen or another biomolecule. In yet another arrangement, the kit can comprise components for performing a nucleic acid probe procedure, wherein the kit comprises one or more sample plates as described herein; and a plurality of beads coated with a nucleic acid, such as a DNA or RNA probe or sequence.

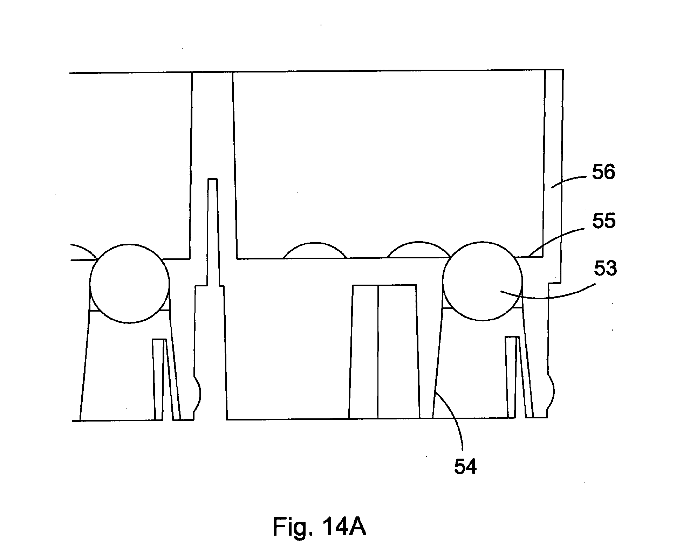

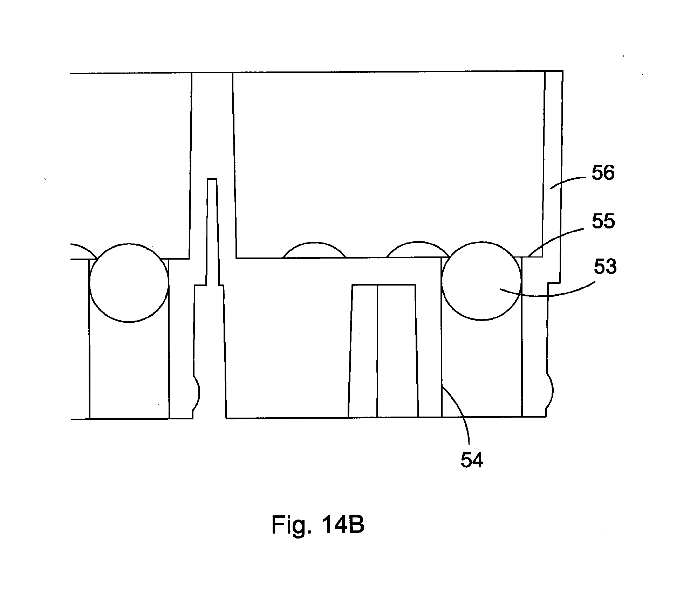

According to the arrangement shown in Fig. 14A reagent beads 53 are loaded into a sample plate from the underneath or rear side of the sample plate. The sample plate comprises a bore or through hole 54 which as shown in Fig. 14A is tapered. However, as will be discussed below, it is also contemplated that the bore or through hole may not be tapered and may instead comprise a substantially cylindrical through hole or bore 54 which has a substantially constant cross-sectional diameter and/or area and/or profile. Fig. 14B shows a sample plate according to an embodiment of the present invention wherein reagent beads or microspheres are secured within a cylindrical bore or through hole 54. The reagent beads or microspheres may be inserted into the cylindrical bore or through hole 54 either from the top or from the bottom. The reagent beads or microspheres are preferably secured within the bore or through hole 54 by an interference fit and the reagent beads or microspheres make a substantially fluid-tight seal around a full circumference of, perimeter of or closed loop around the reagent bead or microsphere.

According to an arrangement, bores or through holes 54 in a sample well may taper from a first diameter at the lowermost part or bottom of the base portion 55 of the sample well 56 to a second narrower diameter towards the uppermost part or top of the base portion 55. The uppermost part or top of the base portion 55 is that part of the base portion 55 which preferably comes into contact with sample fluid in use.

At the top of the bore or through hole 54 immediately below the portion of the base portion 55 which comes into contact with sample fluid, the bore or through hole 54 may be shaped so as to form a tight fit with a reagent bead 53. The uppermost portion of the bore or through hole may comprise a part spherical profile, bulbous region, curved portion or concave region so that a reagent bead 53 which is inserted into the bore or through hole 54 from the underneath of the sample plate fits tightly within the part spherical profile, bulbous region, curved portion or concave region at the top of the bore or through hole 54 as shown in Fig. 14A.

At least a portion of the reagent bead 53 may be arranged to project into the base or bottom of the sample well to form, in effect, part of the base portion of the sample well 56. As a result, the top portion of the reagent bead 53 (above the region where the bead forms a fluid-tight circumferential seal with the wall of the through hole) is arranged so as to come into contact with sample fluid in use. The reagent bead 53 forms a fluid tight seal around the full circumference of the bead 53 with the part spherical profile, bulbous region, curved portion or concave region of the bore or through hole 54.

According to the preferred embodiment macro sized beads 53 are fitted into a sample well 56 of a sample plate so that only the top or upper portion of the reagent bead 53 is exposed to fluid. It should be noted that the luminescent reading process is a 2D operation and only takes into account signal from the visible portion of the reagent bead 53 facing the camera.

According to the preferred embodiment the multiplex well together with reagent beads loaded into the through holes preferably mimics the well established microplate ELISA type of process. The multiplex well according to the preferred embodiment is preferably substantially similar in format to a microplate well,

One of the major factors in processing an ELISA test in a microplate is the efficiency or cleanliness of each step. Any residual fluid from the steps can have an overall effect on the performance of the test e.g. if the conjugate is not completely removed by washing, then residual conjugate will produce a false signal on the bead. This will drive down the sensitivity of the test by increasing the background signal.

The key to efficient processing of the test is not to have any fluid traps in the well. Any corners, pockets or undercuts may trap fluid thereby reducing the performance of the sample plate. The sample plate according to the preferred embodiment allows efficient washing, mixing and aspirating in a similar manner to a conventional microplate well and preferably does not suffer from the problem of trapping fluid.

An advantage of the preferred embodiment is that beads 53 are preferably fitted at a uniform height in a sample well 56 which preferably ensures that each bead 53 is treated identically. Each bead 53 makes a fluid tight sealed fit in the locating detail of a through hole to ensure that there is no fluid trapped under or below the bead 53.

The through hole 54 may comprise a tapered conical hole in which the bead locks into the hole as shown in Fig. 14A or the through hole 54 may comprise a cylindrical undersized hole into which a bead is mechanically pressed into as shown in Fig. 14B. This achieves the goal of preventing fluid going past the bead 53 and becoming trapped underneath or below the bead 53.

If the sample plate comprises one or more tapered through holes 54 as shown in Fig. 14A then the through holes are preferably manufactured with a high degree of accuracy and consistency to ensure that beads are secured within the sample plate at a uniform height (since the reagent beads 53 are preferably pressed into the through holes 54 with a set force and not to a set height). The preferred embodiment of using undersized cylindrical through holes as shown in Fig. 14B does not need to be manufactured to so such a high degree of accuracy since the reagent beads 53 are preferably pressed in to the through holes to a set height and not with a set force.

In some of the arrangements described above reagent beads may be fitted into a blind pocket detail in a sample well i.e. into a closed recess. However, more preferably, a sample plate having through holes in the base portion may be provided as shown and described above with reference to Figs. 14A and 14B.

The assembly of a sample plate which is loaded with reagent beads during production or manufacture should preferably be subjected to a quality control check to ensure that all the beads are sealed to the sample plate. Beads which are loaded into blind pockets as described above will ensure that fluid will not leak out of the well. However, fluid might still leak under the bead and such a leak would be difficult to detect.

According to the preferred embodiment, a sample plate comprising through holes as shown in Fig. 14B allows a pressure check to be carried out as part of the bead to plate assembly, manufacture and quality control checks. This ensures that the bead to plate seal is good. A defective bead or damaged hole would show up as a fall In the manufacture and not when the user runs the test.

The sample plate according to the embodiments as shown in Fig. 14B wherein reagent beads are fitted into the bore from underneath is particularly advantageous for a number of reasons. Firstly, contact between a press in tool and the bead 53 is with the bottom or underneath portion of the reagent bead 53 so any witness mark will also be on the bottom or underneath portion of the reagent bead 53 i.e. not any portion of the reagent bead 53 which will come into contact with sample fluid. Secondly, the top of the through hole 53 in the base portion 55 of the sample well in the example shown in Fig. 14A can be made to match the profile or shape of the reagent bead 53 so that no moat portion is formed around the portion of the bead 53 which protrudes into the base of the sample plate. As a result, the design excludes any possibility of trapping fluid below the reagent bead 53. Thirdly, it does not matter if the tip of a press in tool effectively cross contaminates other beads since the press in tool will only come into contact with the underneath or bottom portion of the reagent beads 53 - the press in tool does not come into contact with the top portion of the reagent beads 53 (i.e. the portion of the reagent beads 53 which will come into contact with sample fluid).

A system for preparing arrays of biomolecules is disclosed in US2009/0069200. Figs. 2 and 3 of US2009/0069200 show spherical reagent beads 9 located in square subwells 8. It is apparent, therefore, that the circular beads placed in the square subwells do not make a fluid-tight seal with the walls of the subwells. The arrangement disclosed in US2009/0069200 also differs from the preferred embodiment of the present invention in that fluid is arranged to pass up through sub wells and over the beads. In contrast, according to the preferred embodiment fluid is only arranged to come into contact with the top surface of a reagent bead 53. According to the preferred embodiment fluid is prevented from passing down a through hole 54 past a reagent bead 53 secured within the through hole 54.

Advantageously, a sample plate according to the preferred embodiment can be cleaned easily during the process steps without trapping fluid under the reagent beads 53. The beads 53 are preferably provided in a format that makes it as close to a cylindrical well as possible and which can also be easily accessed from the top.

The arrangement disclosed in US2009/0069200 uses a common filling chamber or reservoir beneath the beads that is dispensed into in order for the fluid to rise up the individual wells. Circular beads are lodged in square tapered sub wells i.e. the beads do not make a fluid tight seal with the sub wells. Indeed, the fact that spherical beads are provided in square wells enables fluid to flow up, past and around the beads.

The sample plate as disclosed in US2009/0069200 would need to be manufactured in two separate parts as it would not be possible to mould the sample plate including a reservoir as a single piece. The lower part of the sample plate is shown as comprising a discrete plate bottom 11 which would need to be sealed to the upper section of the sample plate comprising a plurality of wells 7 during the manufacturing process. Each well 7 has to be sealed to the plate bottom 11 to ensure that it does not leak. Therefore, the entire grid face between the lower plate bottom 11 and the upper sample wells 7 has to be sealed reliably. As a result, the manufacture process is relatively complex and prone to manufacturing problems.

The sample plate as disclosed in US2009/0069200 is also particularly complex in respect of fluid flow dynamics. The initial dispensing of fluid into the sample plate has to be carried out by dispensing through one of the sub wells. As a result, fluid must be accurately dispensed into a small target area < 1.7 mm which is substantially smaller than the diameter of a sample well according to the preferred embodiment. Furthermore, once fluid has been dispensed into one of the sub wells then the fluid has to flow into the chamber or reservoir 12 at the bottom of the sample plate before rising up evenly into each of the wells to ensure that all the beads are sufficiently immersed. It will be appreciated, therefore, the fluid dynamics associated with the arrangement disclosed in US2009/0069200 are complex and involve tortuous paths which does not lend itself to reproducible results.

Once the sample or conjugate fluid has been dispensed and has flowed past or over the beads in the arrangement disclosed in US2009/0069200, the fluid must then somehow be removed in a commercial product. However, this is particularly problematic as the only access to the sample plate is from the top. Even if a rectangular vacuum tube were sealed against the top of a well it could not be guaranteed that all fluid in the chamber or reservoir in the bottom of the sample plate would be removed. As a result, it is likely that some fluid residue would be left behind in the reservoir and which could cause a false signal in the well.

It will be appreciated, therefore, that the arrangement disclosed in US2009/0069200 suffers from a number of significant problems.

In contrast, the sample plate according to the preferred embodiment does not suffer from the above mentioned problems and represents a significant improvement over known arrangements such as that disclosed in US2009/0069200.

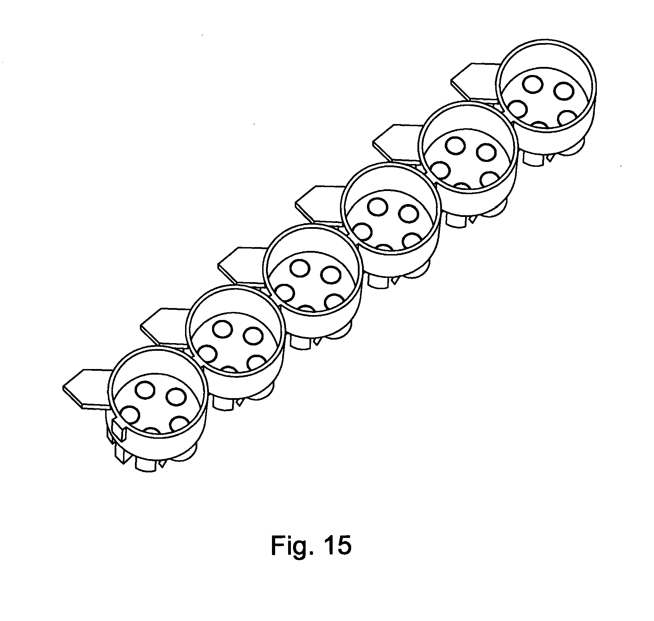

Fig. 15 shows a strip of six sample wells with five 3 mm reagent beads loaded into through holes in each sample well. The reagent beads are loaded into the through holes from the bottom or underneath of the sample plate. The reagent beads are retained within the through holes by upper concave regions formed in the through holes.

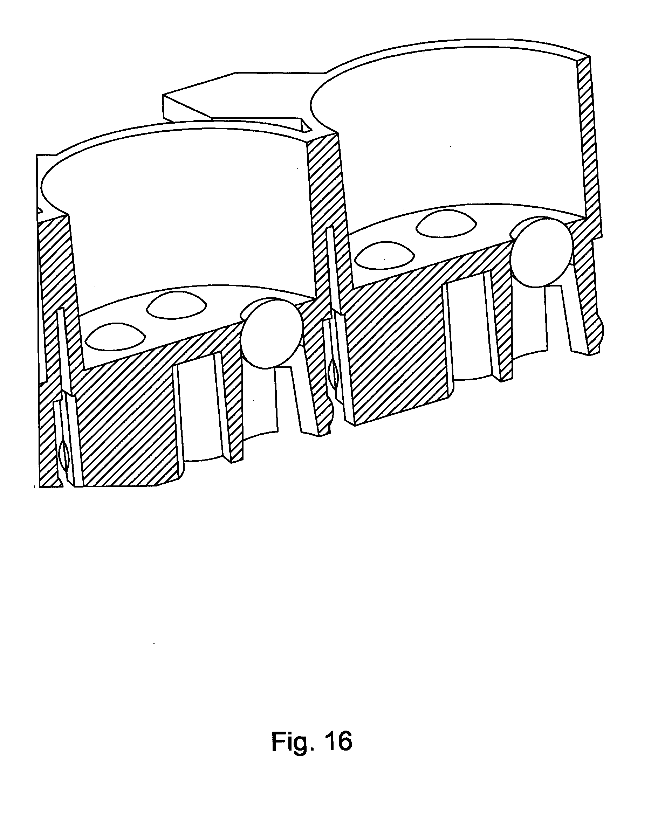

Fig. 16 shows a three dimensional cross-sectional view of the arrangement as shown and described above with reference to Figs. 14A and 15.

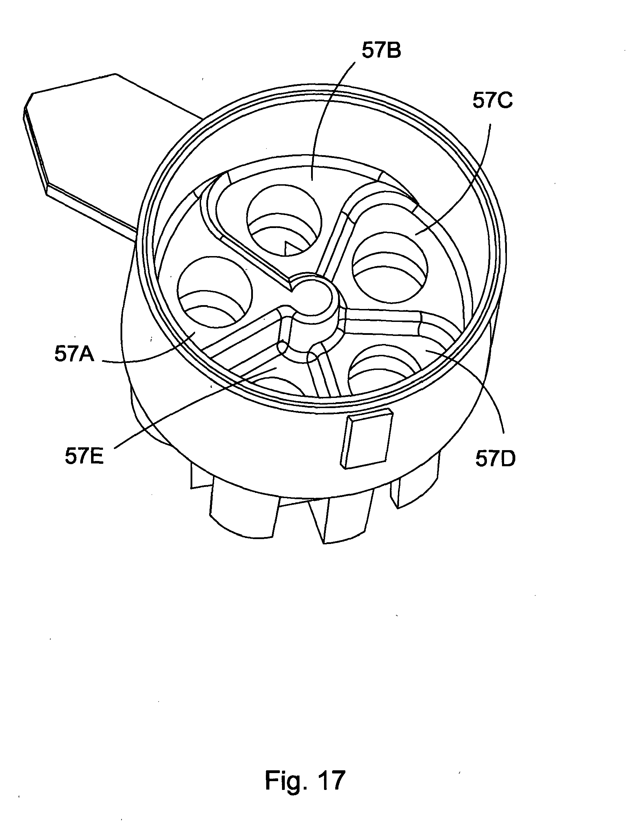

Fig. 17 shows a further embodiment of the present invention wherein the base portion of a sample well is sub-divided into a plurality of segments 57A-E. According to an embodiment each base portion segment 57A-E has one or more open through holes provided in the base portion segment so that a reagent bead can be inserted from above or below into the open through hole. According to another arrangement each base portion segment 57A-E may have one or more blind recesses provided in the base portion segment so that a reagent bead can be inserted from above into the blind recess. According to another embodiment some of the base portion segments 57A-E may comprise one or more through holes and other base portion segments 57A-E may comprise one or more blind recesses. According to the preferred embodiment some or all of the through holes and/or recesses are non-tapered and comprise a cylindrical bore. However, according to a less preferred embodiment some of the through holes and/or some or all of the recesses may be tapered.

According to the preferred embodiment reagent beads or microspheres are retained or secured, in use, within the through holes provided in the base portion segments so as to form a substantially fluid-tight circumferential seal with a wall of the base portion segment which defines the through hole.

The base portion segments 57A-E may be arranged in a spiral or other staggered arrangement in a similar manner to that shown in Fig. 17. The base portion segments 57A-E are preferably arranged at different relative heights to each another so that once reagent beads have been inserted into the open through holes provided in the base portion segments 57A-E then there is preferably no direct line of sight between adjacent reagent beads (or less preferably any line of sight between adjacent reagent beads is significantly reduced). In the embodiment shown in Fig. 17, base portion segment 57A is relatively higher than base portion segment 57B; base portion segment 57B is relatively higher than base portion segment 57C; base portion segment 57C is relatively higher than base portion segment 57D; and base portion segment 57D is relatively higher than base portion segment 57E. Embodiments wherein there is no (or less preferably a reduced) direct line of sight between reagent beads inserted into through holes in base portion segments provided within the same sample well (including the embodiment shown and described above with reference to Fig. 17) are particularly advantageous in that crosstalk between reagent beads is substantially reduced or eliminated when the reagent beads are subsequently optically analysed to determine the intensity of a reaction. According to the preferred embodiment the reagent beads include an indicator which during an analysis step is illuminated by a light source and the intensity of the indicator on a reagent bead is determined by a detector such as a camera to give a measure of the intensity of a reaction.

Figs. 18A and 18B show another embodiment wherein a low-height baffle 58 is provided in a sample well so as to sub-divide the base portion of the sample well into a first base portion 59 having two open through holes and a second base portion 60 having three open through holes. It will be understood that other embodiments are contemplated wherein the first base portion 59 and/or the second base portion 60 may comprise a greater or lesser number of open through holes.

Figs. 19A and 19B show another embodiment wherein a low-height baffle 61 is provided in the sample well. According to the particular embodiment shown in Figs. 19A and 19B, the baffle 61 sub-divides the base portion of the sample well into a first base portion 62A having four open through holes and a second base portion 62B also having four open through holes.

Although the present invention has been described with reference to preferred embodiments, it will be understood by those skilled in the art that various changes in form and detail may be made without departing from the scope of the invention as set forth in the accompanying claims.

## Claims

1. A sample plate comprising one or more sample wells, wherein one or more of said sample wells comprise:
a base portion; and
one or more open through holes provided in said base portion;
**characterised in that**:
said one or more open through holes have a circular cross-sectional shape or profile; and
a reagent bead or microsphere is substantially retained or secured within said through hole by an interference or friction fit so as to form a substantially fluid-tight circumferential seal with a wall of said base portion which defines said through hole;
wherein said open through hole is cylindrical and has a diameter less than a diameter of the reagent bead or microsphere deposited in said through hole so that said reagent bead or microsphere is retained or secured within said through hole by said interference or friction fit.

2. A sample plate as claimed in claim 1, wherein in at least one sample well the base portion comprises a plurality of open through holes and wherein at least some of said plurality of open through holes are arranged so that there is no direct line of sight between reagent beads retained or secured in adjacent open through holes.

3. A sample plate as claimed in claim 1 or 2, wherein in at least one sample well the base portion comprises a plurality of open through holes and wherein said base portion is segmented into a plurality of segments which are arranged at different heights relative to each other.

4. A sample plate as claimed in claim 1, 2 or 3, wherein in at least one sample well the base portion comprises a plurality of open through holes and wherein said sample well further comprises one or more baffles or dividers which separates or divides said base portion into at least a first region and a second region.

5. A sample plate as claimed in claim 4, wherein said one or more baffles or dividers are arranged so as:
(i) to attenuate or eliminate light reflected off one or more reagent beads located in said first region from impinging upon one or more reagent beads located in said second region; and/or
(ii) to attenuate or eliminate light reflected off one or more reagent beads located in said second region from impinging upon one or more reagent beads located in said first region.

6. A method comprising:
providing a sample plate comprising one or more sample wells, wherein one or more of said sample wells comprise a base portion and one or more open through holes provided in said base portion, **characterised in that** said one or more open through holes have a circular cross-sectional shape or profile; and
retaining or securing a reagent bead or microsphere within said through hole by an interference or friction fit so as to form a substantially fluid-tight circumferential seal with a wall of said base portion which defines said through hole;
wherein said open through hole is cylindrical and has a diameter less than a diameter of a reagent bead or microsphere deposited in said through hole so that said reagent bead or microsphere is retained or secured within said through hole by said interference or friction fit.

7. A method of using a sample plate to analyse a sample for multiple analytes comprising:
providing a sample plate as claimed in any of claims 1-5; and
adding a sample to said sample well.

8. A method of using an Enzyme Linked ImmunoSorbent Assay (ELISA) to detect an antigen or an antibody in a sample comprising:
providing a sample plate as claimed in any of claims 1-5; and
adding a sample to said sample well.

9. A method of using a nucleic acid probe to detect a DNA or RNA sequence in a sample comprising:
providing a sample plate as claimed in any of claims 1-5; and
adding a sample to said sample well.

## Patentansprüche

1. Probenplatte, umfassend ein oder mehrere Probenwells, wobei eines oder mehrere der Probenwells umfassen:
einen Basisabschnitt; und
ein oder mehrere offene Durchgangslöcher, die in dem Basisabschnitt bereitgestellt sind;
**dadurch gekennzeichnet, dass**:
das eine oder die mehreren offenen Durchgangslöcher eine kreisförmige Querschnittsform oder ein kreisförmiges Querschnittsprofil aufweisen; und
eine Reagenzperle oder -mikrokugel in dem Durchgangsloch durch Presspassung oder Reibungsschluss im Wesentlichen zurückgehalten oder gesichert ist, um eine im Wesentlichen fluiddichte Umfangsdichtung mit einer Wand des Basisabschnitts, die das Durchgangsloch definiert, zu bilden;
wobei das offene Durchgangsloch zylindrisch ist und einen Durchmesser aufweist, der kleiner als ein Durchmesser der Reagenzperle oder -mikrokugel ist, die in dem Durchgangsloch deponiert ist, so dass die Reagenzperle oder -mikrokugel in dem Durchgangsloch durch die Presspassung oder den Reibungsschluss zurückgehalten oder gesichert ist.

2. Probenplatte gemäß Anspruch 1, bei der bei mindestens einem Probenwell der Basisabschnitt eine Vielzahl von offenen Durchgangslöchern umfasst und wobei mindestens einige der Vielzahl von offenen Durchgangslöchern so angeordnet sind, dass es keine direkte Sichtlinie zwischen in benachbarten offenen Durchgangslöchern zurückgehaltenen oder gesicherten Reagenzperlen gibt.

3. Probenplatte gemäß Anspruch 1 oder 2, bei der in mindestens einem Probenwell der Basisabschnitt eine Vielzahl von offenen Durchgangslöchern umfasst und bei der der Basisabschnitt in eine Vielzahl von Segmenten segmentiert ist, die auf unterschiedlichen Höhen zueinander angeordnet sind.

4. Probenplatte gemäß Anspruch 1, 2 oder 3, bei der in mindestens einem Probenwell der Basisabschnitt eine Vielzahl von offenen Durchgangslöchern umfasst und bei der das Probenwell ferner eine oder mehrere Abschirmungen oder Teiler aufweist, die den Basisabschnitt in mindestens eine erste Region und eine zweite Region auftrennt oder aufteilt.

5. Probenplatte gemäß Anspruch 4, bei der die mindestens eine oder die mehreren Abschirmungen oder Teiler so angeordnet sind,
(i) Licht, das von einer oder von mehreren Reagenzperlen in der ersten Region reflektiert wird, dagegen zu dämpfen oder zu eliminieren, dass es auf eine oder mehrere Reagenzperlen auftrifft, die in der zweiten Region lokalisiert sind; und/oder
(ii) Licht, das von einer oder von mehreren Reagenzperlen in der zweiten Region reflektiert wird, dagegen zu dämpfen oder zu eliminieren, dass es auf eine oder mehrere Reagenzperlen auftrifft, die in der ersten Region lokalisiert sind.

6. Verfahren, umfassend:
Bereitstellen einer Probenplatte, die ein oder mehrere Probenwells umfasst, wobei eines oder mehrere der Probenwells einen Basisabschnitt und einen oder mehrere offene Durchgangslöcher, die in dem Basisabschnitt bereitgestellt sind, umfasst, **dadurch gekennzeichnet, dass** das eine oder die mehreren offenen Durchgangslöcher eine kreisförmige Querschnittsform oder ein kreisförmiges Querschnittsprofil aufweisen; und
Zurückhalten oder Sichern einer Reagenzperle oder - mikrokugel in dem Durchgangsloch durch Presspassung oder Reibungsschluss, um eine im Wesentlichen fluiddichte Umfangsdichtung mit einer Wand des Basisabschnitts, die das Durchgangsloch definiert, zu bilden;
wobei das offene Durchgangsloch zylindrisch ist und einen Durchmesser aufweist, der kleiner ist als ein Durchmesser einer Reagenzperle oder -mikrokugel, die in dem Durchgangsloch deponiert ist, so dass die Reagenzperle oder -mikrokugel in dem Durchgangsloch durch die Presspassung oder den Reibungsschluss zurückgehalten oder gesichert ist.

7. Verfahren zum Verwenden einer Probenplatte zum Analysieren einer Probe auf mehrere Analyte, umfassend:
Bereitstellen einer Probenplatte, so wie sie in einem der Ansprüche 1 - 5 beansprucht ist; und
Hinzugeben einer Probe in das Probenwell.

8. Verfahren zum Verwenden eines Enzyme Linked ImmunoSorbent Assays (ELISA), um ein Antigen oder einen Antikörper in einer Probe zu ermitteln, umfassend:
Bereitstellen einer Probenplatte, so wie sie in einem der Ansprüche 1 - 5 beansprucht ist; und
Hinzugeben einer Probe in das Probenwell.

9. Verfahren zum Verwenden einer Nukleinsäuresonde, um eine DNA-oder RNA-Sequenz in einer Probe zu ermitteln, umfassend:
Bereitstellen einer Probenplatte, so wie sie in einem der Ansprüche 1 - 5 beansprucht ist; und
Hinzugeben einer Probe in das Probenwell.

## Revendications

1. Plaque d'échantillon comprenant un ou plusieurs puits d'échantillon, dans laquelle un ou plusieurs desdits puits d'échantillon comprend ou comprennent :
une portion de base ; et
un ou plusieurs trous traversants ouverts ménagés dans ladite portion de base ;
**caractérisée en ce que** :
lesdits un ou plusieurs trous traversants ouverts a ou ont une forme ou un profil circulaire en coupe transversale ; et
une bille ou une microsphère de réactif est sensiblement retenue ou immobilisée dans ledit trou traversant par un joint à ajustement serré ou à frottement de manière à former un joint d'étanchéité circonférentiel sensiblement étanche aux fluides avec une paroi de ladite portion de base qui définit ledit trou traversant ;
dans laquelle ledit trou traversant ouvert est cylindrique et a un diamètre inférieur au diamètre de la bille ou microsphère de réactif déposée dans ledit trou traversant de sorte que ladite bille ou microsphère de réactif soit retenue ou immobilisée dans ledit trou traversant par ledit joint à ajustement serré ou à frottement.

2. Plaque d'échantillon selon la revendication 1, dans laquelle, dans au moins un puits d'échantillon, la portion de base comprend une pluralité de trous traversants ouverts et dans laquelle au moins certains de ladite pluralité de trous traversants ouverts sont ménagés de sorte qu'il n'y ait pas de ligne de vision directe entre les billes de réactif retenues ou immobilisées dans des trous traversants ouverts adjacents.

3. Plaque d'échantillon selon la revendication 1 ou la revendication 2, dans laquelle, dans au moins un puits d'échantillon, la portion de base comprend une pluralité de trous traversants ouverts et dans laquelle ladite portion de base est segmentée en une pluralité de segments qui sont aménagés à différentes hauteurs l'un par rapport à l'autre.

4. Plaque d'échantillon selon la revendication 1, 2 ou 3, dans laquelle, dans au moins un puits d'échantillon, la portion de base comprend une pluralité de trous traversants ouverts et dans laquelle ledit puits d'échantillon comprend une ou plusieurs chicanes ou un ou plusieurs diviseurs qui séparent ou divisent ladite portion de base en au moins une première région et une seconde région.

5. Plaque d'échantillon selon la revendication 4, dans laquelle lesdites une ou plusieurs chicanes ou lesdits un ou plusieurs diviseurs sont aménagés de manière :
(i) à atténuer ou éliminer la lumière réfléchie par une ou plusieurs billes de réactif situées dans ladite première région pour l'empêcher de heurter une ou plusieurs billes de réactif situées dans ladite seconde région ; et/ou
(ii) à atténuer ou éliminer la lumière réfléchie par une ou plusieurs billes de réactif situées dans ladite seconde région pour l'empêcher de heurter une ou plusieurs billes de réactif situées dans ladite première région.

6. Procédé comprenant les étapes consistant à :
fournir une plaque d'échantillon comprenant un ou plusieurs puits d'échantillon, dans lequel un ou plusieurs desdits puits d'échantillon comprend ou comprennent une portion de base et un ou plusieurs trous traversants ouverts ménagés dans ladite portion de base, **caractérisé en ce que** lesdits un ou plusieurs trous traversants ouverts ont une forme ou un profil circulaire en coupe transversale ; et
retenir ou immobiliser une bille ou microsphère de réactif dans ledit trou traversant par un joint à ajustement serré ou par frottement de façon à former un joint d'étanchéité circonférentiel sensiblement étanche aux fluides avec une paroi de ladite portion de base qui définit ledit trou traversant ;
dans lequel ledit trou traversant ouvert est cylindrique et a un diamètre inférieur au diamètre d'une bille ou microsphère de réactif déposée dans ledit trou traversant de sorte que ladite bille ou microsphère de réactif soit retenue ou immobilisée dans ledit trou traversant par ledit joint à ajustement serré ou par frottement.

7. Procédé d'utilisation d'une plaque d'échantillon pour analyser un échantillon pour de multiples analytes, comprenant les étapes consistant à :
fournir une plaque d'échantillon selon l'une quelconque des revendications 1 à 5 ; et
ajouter un échantillon audit puits d'échantillon.

8. Procédé d'utilisation d'un dosage par la méthode ELISA pour détecter un antigène ou un anticorps dans un échantillon, comprenant les étapes consistant à :
fournir une plaque d'échantillon selon l'une quelconque des revendications 1 à 5 ; et
ajouter un échantillon audit puits d'échantillon.

9. Procédé d'utilisation d'une sonde d'acide nucléique pour détecter une séquence d'ADN ou d'ARN dans un échantillon, comprenant les étapes consistant à :
fournir une plaque d'échantillon selon l'une quelconque des revendications 1 à 5 ; et
ajouter un échantillon audit puits d'échantillon.
